# EUROPEAN PATENT APPLICATION

(11) **EP 3 207 932 A1**
(43) Date of publication of application: **23.08.2017**
(21) Application number: 16156521.3
(22) Date of filing: 19.02.2016
(51) Int. Cl.: A61K 31/7068, A61P 25/00, A61K 31/7042, A61K 31/02, A61K 31/145, A61K 31/166, A61K 31/198, A61K 31/245, A61K 31/35, A61K 31/352, A61K 31/381, A61K 31/405, A61K 31/4188, A61K 31/473, A61K 31/502, G01N 33/00

(54) **DNA METHYLTRANSFERASE INHIBITORS FOR RETT SYNDROME THERAPY**

(71) Applicant: Universität Stuttgart, 70174 Stuttgart (DE)
(72) Inventor: JELTSCH, Albert, 71277 Rutesheim (DE); JURKOWSKA, Renata, 71032 Böblingen (DE); RAJAVELU, Arumugam, 636807 Tamil Nadu (IN); LUNGU, Cristiana, 73776 Altbach (DE)
(74) Representative: PATERIS Patentanwälte PartmbB

(57) **Abstract**

The invention discloses a DNA methyltransferase inhibitor, or a pharmaceutically acceptable salt thereof, for use in treating Rett syndrome. Further disclosed is a pharmaceutical composition comprising a DNA methyltransferase inhibitor, or a pharmaceutically acceptable salt thereof, and a pharmaceutically acceptable carrier for use in treating Rett syndrome. Further disclosed is an *in vitro* method for diagnosing Rett syndrome in a subject comprising the step of analysing DNA methylation in a sample of the subject. Further disclosed is a kit for diagnosing Rett syndrome in a subject *in vitro* comprising reagents for quantifying a level of DNA methylation in a sample of the subject, wherein an increased level of DNA methylation in the sample compared to a reference value obtained from a reference sample of a healthy individual indicates that the subject has Rett syndrome.

## Description

### Field of the invention

The invention relates to a DNA methyltransferase inhibitor, or a pharmaceutically acceptable salt thereof, for use in treating Rett syndrome. The invention further relates to a pharmaceutical composition for use in treating Rett syndrome. Additionally, the invention relates to an *in vitro* method for diagnosing Rett syndrome in a subject. The invention further relates to a kit for diagnosing Rett syndrome in a subject *in vitro.*

### Background of the invention

Rett syndrome is a rare genetic disease that almost exclusively affects females. It is a postnatal neurological disorder and occurs in 1 of every 10,000 female births worldwide. Rett syndrome symptoms appear after an early period of apparently normal or near normal development until six to eighteen months of life. Symptoms are similar to autism and include repetitive hand movements such as wringing and problems with learning, speaking, walking, breathing, sensory sensations, mood and cardiac function. Chewing, swallowing and digestion may also be affected. The severity of the symptoms varies from child to child.

Rett syndrome is caused by mutations in the methyl CpG binding protein 2 (MECP2) gene which is located on the X chromosome. In at least 95% of Rett syndrome cases, the mutation is a *de novo* mutation in the child. The MeCP2 protein is a nuclear protein with a methyl-CpG-binding domain (MBD domain) that specifically binds to methylated DNA. In addition to the MBD domain, MeCP2 has a transcriptional repression domain (TRD domain), which serves as a protein recruitment platform and shows methylation-independent DNA binding. MeCP2 is known to act as a repressor of gene transcription, but it may also function as a transcriptional activator. The MeCP2 protein is found in all cells in the body, but is highly expressed in neurons. MeCP2 plays an essential role in brain plasticity. It also functions as a structural protein in neurons and forms a specific type of chromatin which is depleted of histone H1. Many of the mutations found in Rett syndrome patients are associated with a reduced or absent expression of the MECP2 gene or lead to an inactive version of the MeCP2 protein. As a result, the normal functioning of nerve cells is impaired.

Rett syndrome is diagnosed by observing signs and symptoms during the child's early growth and development. Main diagnostic criteria include partial or complete loss of acquired purposeful hand skills, partial or complete loss of acquired spoken language, repetitive hand movements such as hand wringing or squeezing, clap-ping or rubbing, and gait abnormalities such as toe-walking or an unsteady, wide-based, stiff-legged walk. In addition, a test to confirm the presence of a mutation in the MECP2 gene is usually performed.

Therapies of Rett syndrome include occupational therapy, speech-language therapy and physiotherapy. Certain complications such as scoliosis, gastrointestinal and cardiac malfunctions can be treated by corrective surgery, nutritional supplements and/or respective medicaments such as beta-blockers depending on their severity. Treatment by restoring MECP2 gene function is being investigated, but is still far from clinical application.

Therefore, additional means for treating Rett syndrome are needed.

### Summary of the invention

In a first aspect, the invention relates to a DNA methyltransferase inhibitor, or a pharmaceutically acceptable salt thereof, for use in treating Rett syndrome.

In a further aspect, the invention relates to a pharmaceutical composition comprising a DNA methyltransferase inhibitor, or a pharmaceutically acceptable salt thereof, and a pharmaceutically acceptable carrier for use in treating Rett syndrome.

In a further aspect, the invention relates to an *in vitro* method for diagnosing Rett syndrome in a subject comprising the step of analysing DNA methylation in a sample of the subject.

In a further aspect, the invention relates to a kit for diagnosing Rett syndrome in a subject *in vitro* comprising reagents for quantifying a level of DNA methylation in a sample of the subject, wherein an increased level of DNA methylation in the sample compared to a reference value obtained from a reference sample of a healthy individual indicates that the subject has Rett syndrome.

### Brief description of the drawings

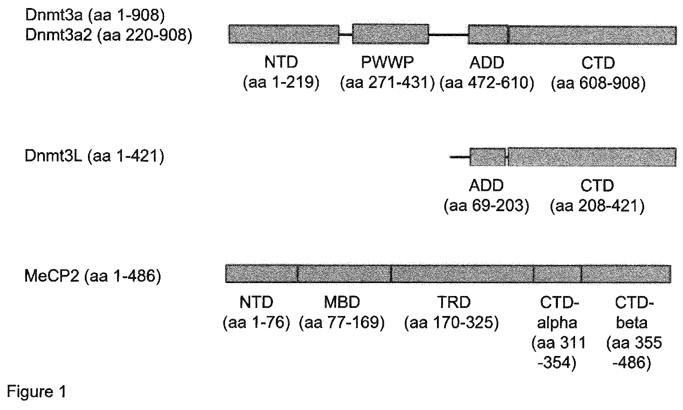

Figure 1 shows the protein domain structures of mouse Dnmt3a, mouse Dnmt3L and human MeCP2 isoform 1 proteins as well as the domain boundaries used in the examples (aa, amino acids; ADD, ATRX-DNMT3-DNMT3L domain; CTD, C-terminal domain; MBD, methyl-CpG-binding domain; NTD, N-terminal domain; TRD, transcriptional repression domain).

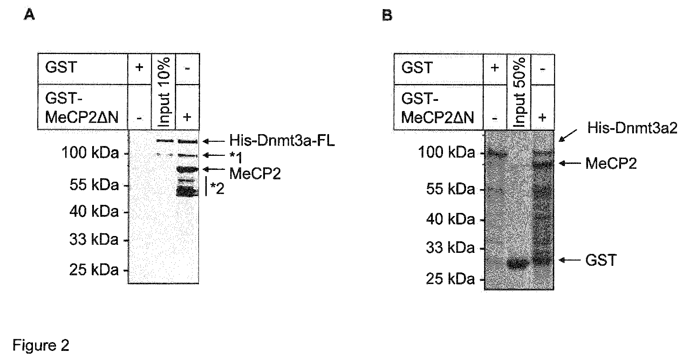

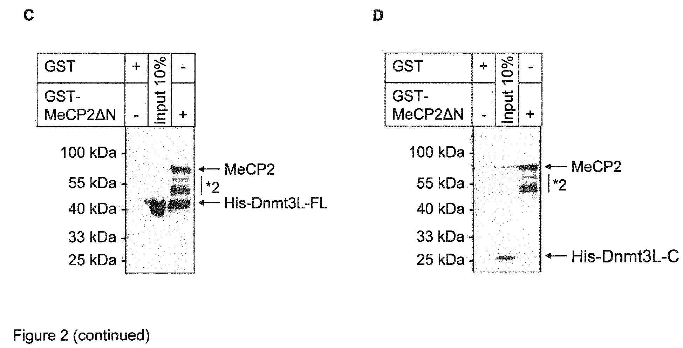

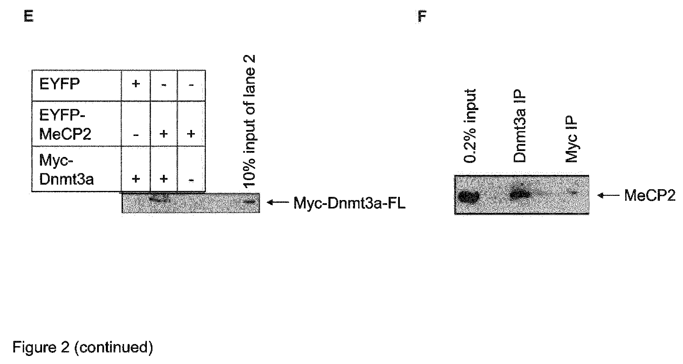

Figure 2 shows the results of GST pull-down experiments with Dnmt3 and MeCP2 proteins. A) Western blot analyses using anti-His antibody of pull-down of His-Dnmt3a by GST-tagged N-terminal truncated MeCP2 (MeCP2ΔN, amino acid residues 104-486). MeCP2 carries an endogenous His-tag. The bands labelled with *1 and *2 correspond to degraded Dnmt3a and MeCP2ΔN, respectively. FL, full-length. B) SDS-PAGE stained with Coomassie Brilliant Blue of pull-down of His-Dnmt3a2 by GST-tagged MeCP2ΔN. C) Western blot analyses using anti-His antibody of pull-down of His-Dnmt3L by GST-tagged MeCP2ΔN. D) Western blot analyses using anti-His antibody of pull-down of His-tagged C-terminal domain of Dnmt3L (Dnmt3L-C) by GST-tagged MeCP2ΔN showing absence of interaction. E) Western blot analyses using anti-Myc antibody of pull-down of Myc-tagged Dnmt3a by EYFP-tagged MeCP2 after transient co-expression of both proteins in human HEK293 cells. FL, full-length. F) Western blot analyses using anti-MeCP2 antibody of pull-down of endogenous MeCP2 by immunoprecipitation of endogenous Dnmt3a (Dnmt3a IP) using mouse brain protein extracts. An immunoprecipitation using anti-Myc antibody (Myc IP) was conducted as control.

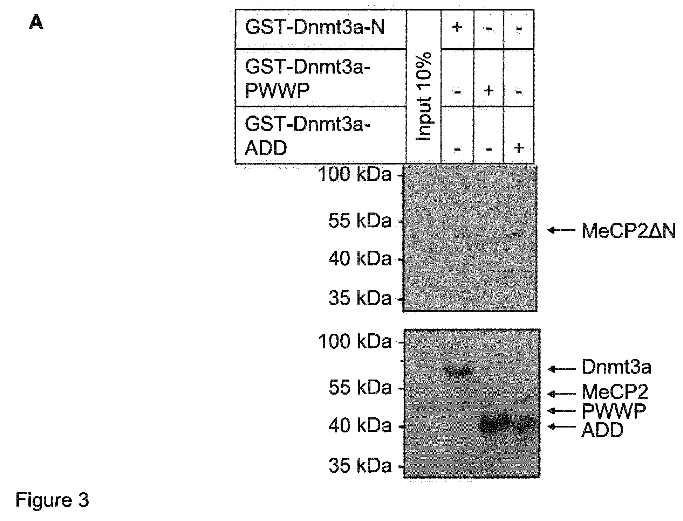

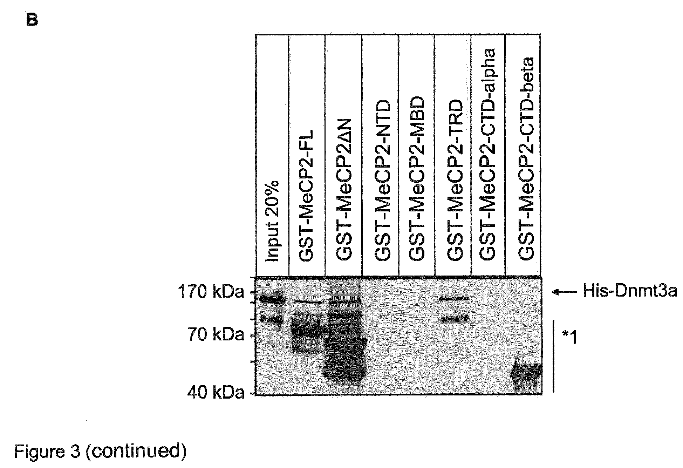

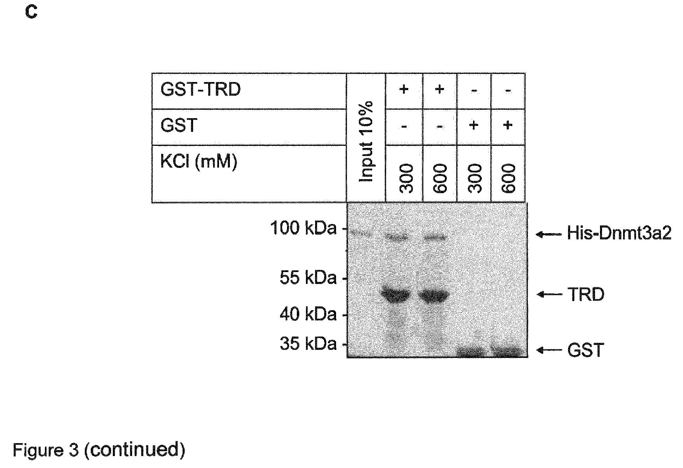

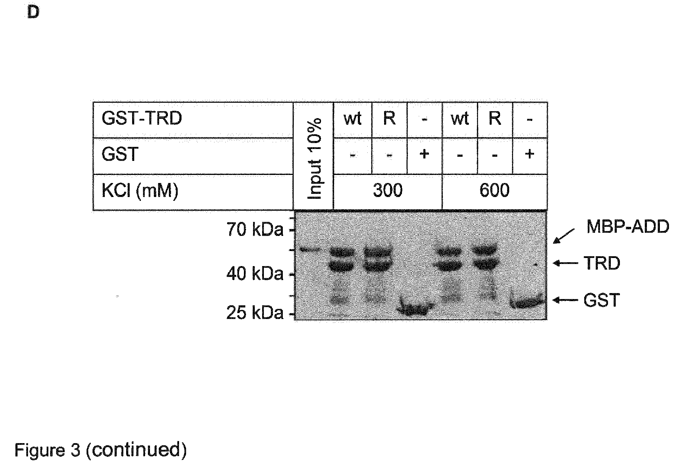

Figure 3 shows the results of mapping analyses of the Dnmt3a-MeCP2 interaction. A) Western blot analyses using anti-His antibody of pull-down of MeCP2ΔN with different GST-tagged Dnmt3a domains, showing an interaction with the ADD domain. Below the Western blot, the respective Ponceau S stain is shown. B) Western blot analyses using anti-His antibody of pull-down of Dnmt3a with different GST-tagged MeCP2 domains showing interaction with the TRD domain. MeCP2 contains an endogenous His-tag in its CTD-beta domain. *1 corresponds to MeCP2 proteins and domains. C) SDS-PAGE stained with Coomassie Brilliant Blue of pull-down of His-Dnmt3a2 with GST-tagged MeCP2 TRD domain. D) SDS-PAGE stained with Coomassie Brilliant Blue of pull-down of MBP-tagged ADD domain by GST-tagged MeCP2 TRD domain (wt, wild-type) or MeCP2 TRD domain containing the R306C Rett mutation (R).

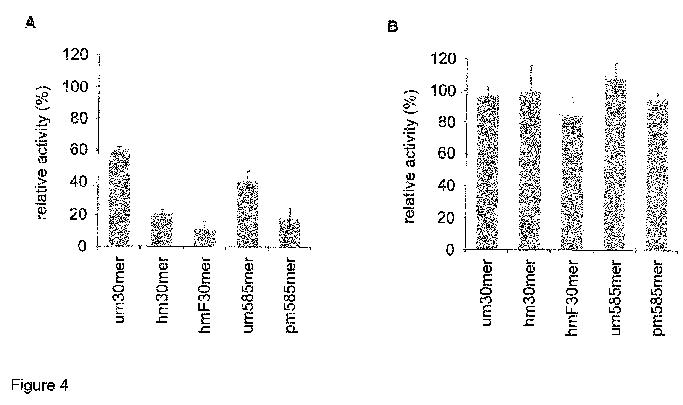

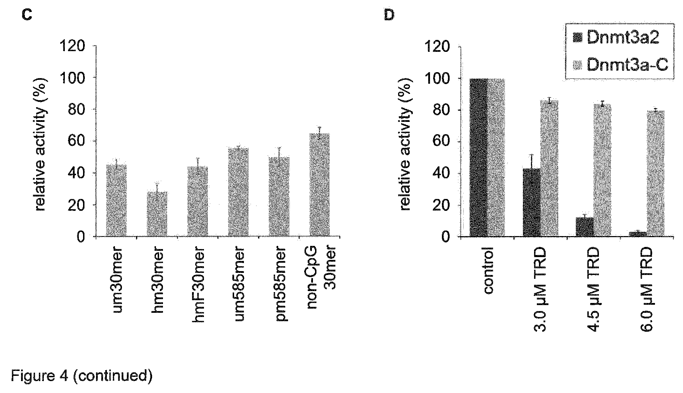

Figure 4 shows the inhibition of Dnmt3a2 by addition of MeCP2 (A and B) or MeCP2 TRD domain (C and D). The following substrates for DNA methylation were used: um30mer, unmethylated 30mer; hm30mer, hemimethylated 30mer; hmF30mer, hemimethylated 30mer with optimized flanks; um585mer, unmethylated 585mer; pm585mer, partially methylated 585mer; non-CpG 30mer. In A) Dnmt3a2 was used and MeCP2 was added. In B) Dnmt3a-C was used and MeCP2 was added. In C) Dnmt3a2 was used and MeCP2 TRD domain was added. Identical control reactions without addition of MeCP2 were used to calculate relative activities. D) Inhibition of Dnmt3a2 by addition of increasing concentrations of MeCP2 TRD domain using the um30mer substrate. Control refers to a reaction without added TRD domain. All error bars indicate the standard error of the mean (SEM).

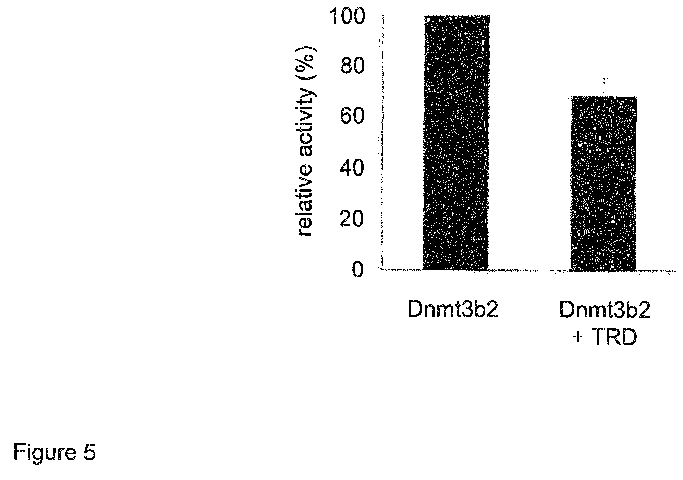

Figure 5 shows the inhibition of Dnmt3b2 for methylation of the um30mer substrate by addition of the MeCP2 TRD domain. The activity of Dnmt3b2 in the absence of the TRD domain was normalized to 100%. The error bar indicates the SEM for three independent experiments.

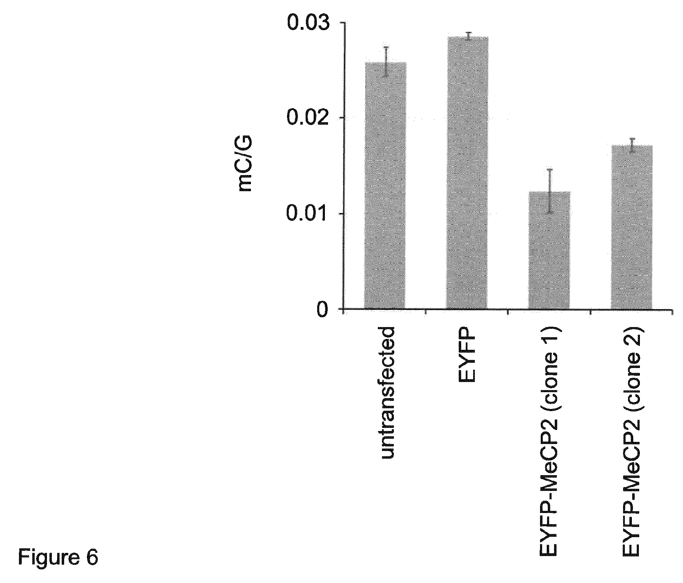

Figure 6 shows the analysis of global DNA methylation levels after expression of MeCP2 in human HCT116 cells having a DNMT1 hypomorphic allele. Different cell lines stably expressing MeCP2 showed an approximately 2-fold reduction of global DNA methylation. The error bars represent the SEM. The amount of methylcytosine (mC) determined by LC-MS is indicated in relation to the amount of guanosine (G) in the sample (mC/G).

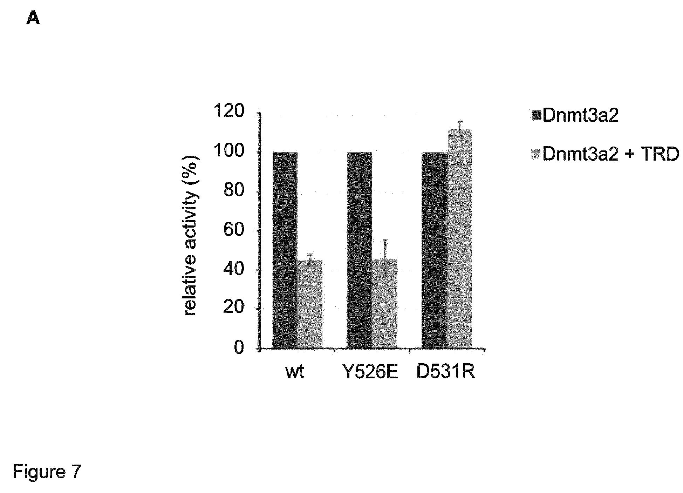

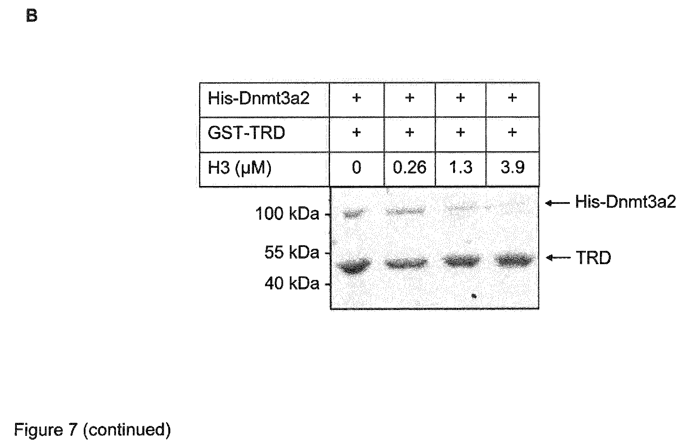

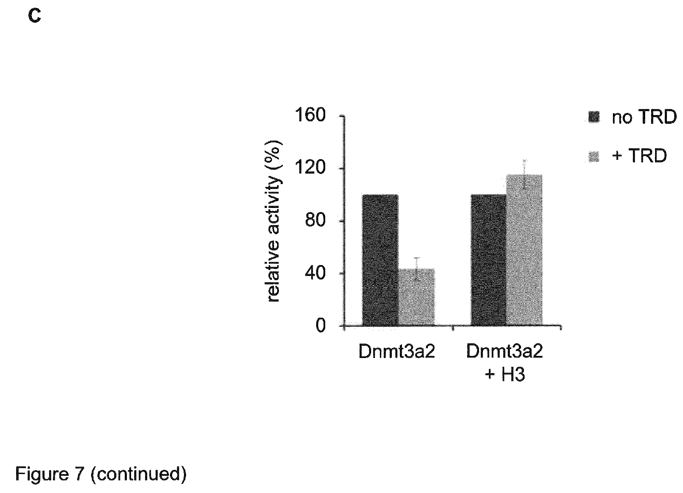

Figure 7 shows studies on the mechanism of the inhibition of Dnmt3a by the TRD domain of MeCP2. A) Inhibition of Dnmt3a2 wild-type (wt) and its conformational variants Y526E and D531R by the MeCP2 TRD domain. The inhibition is lost in the D531 R variant carrying a mutation, which prevents the autoinhibitory conformation. B) SDS-PAGE stained with Coomassie Brilliant Blue of pull-down of His-Dnmt3a2 (0.25 µM) by GST-TRD in the presence of increasing concentrations of recombinant Histone H3 indicating that the H3 binding to Dnmt3a2 interferes with the TRD interaction. C) DNA methylation activities of Dnmt3a2 and Dnmt3a2 bound to H3 peptide (amino acids 1-19, 25 µM) in the absence and presence of TRD (3 µM) showing the loss of TRD inhibition in the presence of H3 peptide.

### Detailed description of the invention

In a first aspect, the invention relates to a DNA methyltransferase inhibitor, or a pharmaceutically acceptable salt thereof, for use in treating Rett syndrome.

The inventors found that the methyl CpG binding protein 2 (MeCP2) interacts with DNA methyltransferase 3 alpha (Dnmt3a) and that the interaction with MeCP2 inhibits the enzymatic activity of Dnmt3a and DNA methyltransferase 3 beta (Dnmt3b). DNA methyltransferases are enzymes that catalyze the transfer of a methyl group from the methyl donor S-adenosylmethionine to the C-5 of cytosine in DNA. DNA methylation plays a key role in embryonic development, genomic imprinting, X chromosome inactivation, cell differentiation and in the regulation of gene transcription. DNA methylation mainly occurs at cytosines within CpG dinucleotides and approximately 80% of CpG dinucleotides are methylated. CpG dinu-cleotides are mainly located in so-called CpG islands which occur at approximately 60% of all gene promoters and in repeated sequences. If a promoter region of a gene has high levels of 5-methylcytosines, transcription of the gene does not take place (so-called gene repression or gene silencing). As a consequence, aberrant DNA methylation patterns are associated with underexpression or over-expression of certain proteins, leading to diverse pathologies such as cancer.

Dnmt3a and Dnmt3b are *de novo* methyltransferases that establish DNA methylation during embryogenesis. Dnmt3a and Dnmt3b comprise two parts, namely a large multi-domain N-terminal part and a C-terminal catalytic domain. The N-terminal part guides the nuclear and sub-nuclear localization of the enzyme and mediates its interactions with other proteins, DNA and chromatin, thereby regulating the catalytic activity. The N-terminal parts of Dnmt3a and Dnmt3b have two defined sub-domains, the PWWP domain and the ATRX-DNMT3-DNMT3L domain (ADD domain). The PWWP domain binds to histone H3 tails methylated at K36 and is essential for the heterochromatic localization of the enzyme and for the methylation of gene bodies. The ADD domain contacts the N-terminus of the histone H3 tail and is sensitive to the modification state of H3K4, with modifications larger than monomethylation precluding binding.

Dnmt3a is highly expressed in neurons and is involved in neuromuscular control, synaptic plasticity, learning and memory.

It was found that MeCP2 directly interacts with Dnmt3a and that the interaction is mediated by the TRD domain of the MeCP2 protein and the ADD domain of the Dnmt3a protein.

A common missense mutation in the MeCP2 protein in Rett syndrome patients is R306C. Although the R306C mutation is located in the TRD domain of MeCP2, the inventors found that it does not affect the interaction of the MeCP2 TRD domain with Dnmt3a. Thus the interaction of MeCP2 and Dnmt3a also occurs in Rett syndrome patients. The same is likely to be the case for Dnmt3b.

The inventors further found that binding of MeCP2 to Dnmt3a or Dnmt3b strongly inhibits the enzymatic activity of Dnmt3a and Dnmt3b, respectively. Using unmethylated DNA substrates, the interaction of MeCP2 with Dnmt3a resulted in a reduction of the enzymatic activity of Dnmt3a of about 40-60%. The interaction of MeCP2 with Dnmt3b resulted in a comparable reduction in the enzymatic activity of Dnmt3b. When methylated DNA substrates were used, the enzymatic activity of Dnmt3a was even further reduced by the interaction with MeCP2, namely by about 80%. This may be due to a better binding of MeCP2 to methylated DNA compared to unmethylated DNA. The reduction of the DNA methylation activity of Dnmt3a was not only observed on CpG dinucleotide sites, but also to a similar extent on non-CpG sites. Accordingly, overexpression of the MeCP2 protein led to a global reduction of DNA methylation in human cells. Regarding the mechanism of the inhibition of Dnmt3a activity, it was found that through the binding of MeCP2 to Dnmt3a, MeCP2 allosterically stabilizes an autoinhibitory conformation of Dnmt3a. The inhibition of Dnmt3b activity is likely to be based on the same mechanism.

Given that both Dnmt3a and MeCP2 are highly expressed in neurons and have important biological functions in the brain, the interaction of MeCP2 with Dnmt3a is likely to play a key role in controlling the level of DNA methylation in the brain.

Taken together, the inventors found that MeCP2 functions as a negative regulator of global DNA methylation of the genome. MeCP2 primarily reduces overall untargeted DNA methylation of the genome and thus functions as a safeguard to prevent aberrant untargeted DNA methylation. Since MeCP2 is either dysfunctional or not expressed in Rett syndrome patients, the repression of the enzymatic activity of Dnmt3a and Dnmt3b is missing. As a result, Dnmt3a and Dnmt3b are hyperactive and the overall level of DNA methylation of the genome is increased compared to healthy individuals. Given the central role of DNA methylation in human cells including neurons, the inventors anticipate that the excessive DNA methylation contributes to the symptoms of Rett syndrome, in particular by causing a global reduction of gene expression. Hence, DNA methyltransferase inhibitors present useful agents for treating Rett syndrome.

DNA methyltransferases are attractive therapeutic targets since DNA methylation is reversible such that an aberrantly increased DNA methylation level can be reverted by inhibition of DNA methyltransferases. By reducing the activity of DNA methyltransferases, the inhibitors will lower the aberrantly increased DNA methylation levels in the genome of Rett syndrome patients. Since DNA methylation plays a key role in the regulation of gene transcription, the treatment with DNA methyltransferase inhibitors will help restoring the level of gene transcription found in healthy individuals. This will ameliorate the symptoms of Rett syndrome.

Inhibiting one or more DNA methyltransferases is a promising strategy for ameliorating several Rett syndrome symptoms at the same time. Thus, it may allow to reduce the number of therapies that are directed to single symptoms only. This will contribute to an overall improvement in the quality of life of Rett syndrome patients.

The term "DNA methyltransferase inhibitor" refers to any compound that reduces the enzymatic activity of a DNA methyltransferase. It includes reversible inhibitors as well as irreversible inhibitors. To date, DNA methyltransferase inhibitors such as 5-azacytidine and 5-aza-2'-deoxycytidine are used in the treatment of leukemia. Due to their potential for cancer therapy in general, the development of new DNA methyltransferase inhibitors and the improvement of known inhibitors are areas of intensive research and several DNA methyltransferase inhibitors are currently being tested in cancer patients in clinical trials. DNA methyltransferase inhibitors are described for example in Liu et al. 2015 and Fahy et al. 2012. Treatment with DNA methyltransferase inhibitors is a type of epigenetic therapy, i.e. a therapy targeting the epigenome. The term "epigenome" refers to the entirety of DNA methylation and histone modification patterns.

The term "pharmaceutically acceptable salt" refers to a salt version of the inhibitor that retains the biological effectiveness of the inhibitor and that may be administered to a subject without undue toxicity or side effects such as irritation, allergic responses or other complications in the amounts used. As many pharmaceutically active compounds, a DNA methyltransferase inhibitor may have certain suboptimal physicochemical or pharmaceutical characteristics that can be overcome by pairing a basic or acidic inhibitor molecule with a counterion in order to obtain a salt version of the inhibitor. The preparation of an inhibitor as a pharmaceutically acceptable salt form may improve, for example, the bioavailability, stability and/or manufacturability of the inhibitor.

The pharmaceutically acceptable salt may be an inorganic salt such as hydrochloride, hydrobromide, phosphate, nitrate, carbonate, bicarbonate and/or sulphate.

The pharmaceutically acceptable salt may be a salt of an organic acid such as acetate, citrate, propionate, malonate, succinate, ascorbate, tartrate and/or benzoate.

In a preferred embodiment, the DNA methyltransferase inhibitor is a nucleoside analogue and/or a non-nucleoside compound. A nucleoside is a nucleobase linked to a 5-carbon sugar which is either ribose or deoxyribose. Examples of nucleosides include cytidine, deoxycytidine, thymidine, deoxythymidine, uridine, adenosine, deoxyadenosine, guanosine, deoxyguanosine and inosine. The term "nucleoside analogue" as used herein refers to a nucleoside with a modified nucleobase and/or a modified sugar. Preferably the nucleoside analogue has a modified nucleobase. Nucleoside analogues need to be incorporated into the DNA to be active DNA methyltransferase inhibitors. Once incorporated into the DNA, they irreversibly form a covalent complex with a DNA methyltransferase. The complex then triggers DNA repair and degradation of the DNA methyltransferase.

The term "non-nucleoside compound" as used herein refers to any DNA methyltransferase inhibitor having a chemical structure different from nucleoside analogues. Non-nucleoside compounds inhibit DNA methyltransferases by variable mechanisms. Most non-nucleoside compounds bind to DNA methyltransferases.

Nucleoside analogues are more effective DNA methyltransferase inhibitors compared to non-nucleoside compounds. Therefore, in a further preferred embodiment, the DNA methyltransferase inhibitor is a nucleoside analogue.

In a preferred embodiment, the nucleoside analogue is selected from the group consisting of
5-azacytidine,
5-aza-2'-deoxycytidine,
5,6-dihydro-5-azacytidine,
5-fluoro-2'-deoxycytidine,
1-(beta-D-ribofuranosyl)-1,2-dihydropyrimidin-2-one,
5-aza-2'-deoxycytidine-p-deoxyguanosine,
fluorocyclopentenylcytosine,
2-(p-nitrophenyl) ethoxycarbonyl-5-aza-2'-deoxycytidine,
CP-4200,
4'-thio-2'-deoxycytidine,
5-aza-4'-thio-2'-deoxycytidine, and
1-beta-D-arabinofuranosyl-5-azacytosine.

All of these nucleoside analogues are cytidine or deoxycytidine analogues. Their chemical structures as well as further information are given in table 1. Alternative names and/or abbreviations of the inhibitors are indicated in square brackets. FDA, US Food and Drug Administration.

**Table 1:**

| **Inhibitor** | **Chemical structure** | **Comments** |
|---|---|---|
| 5-azacytidine [azacitidine, AZA] | 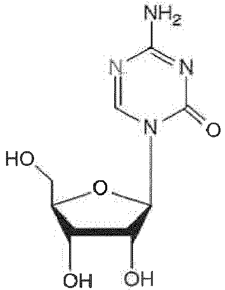 | approved by the FDA for the treatment of leukemia and myelodysplastic syndrome |
| 5-aza-2'-deoxycytidine [decitabine, DAC] | 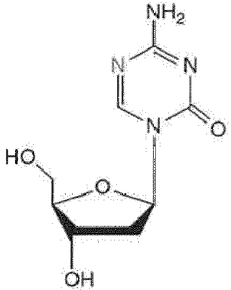 | approved by the FDA for the treatment of leukemia and myelodysplastic syndrome, more active and less secondary effects compared to AZA |
| 5,6-dihydro-5-azacytidine [DHAC] | 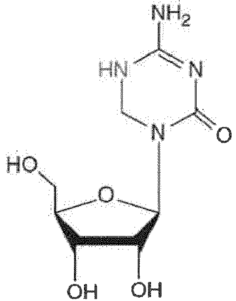 | clinical trials in cancer therapy were stopped (low efficacy) |
| 5-fluoro-2'-deoxycytidine [FdCyd] | 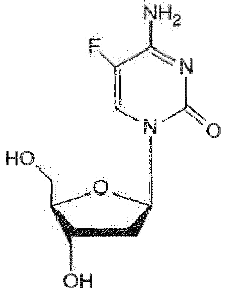 | in clinical trials for cancer therapy |
| 1-(beta-D-ribofuranosyl)-1,2-dihydropyrimidin-2-one [zebularine] | 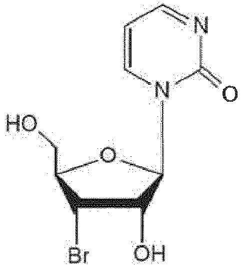 | lower potency, but reduced toxicity and better oral bioavailabilty compared to AZA and DAC |
| 5-aza-2'-deoxycytidine-p-deoxyguanosine [decitabine-p-deoxyguanosine, SGI-110] | 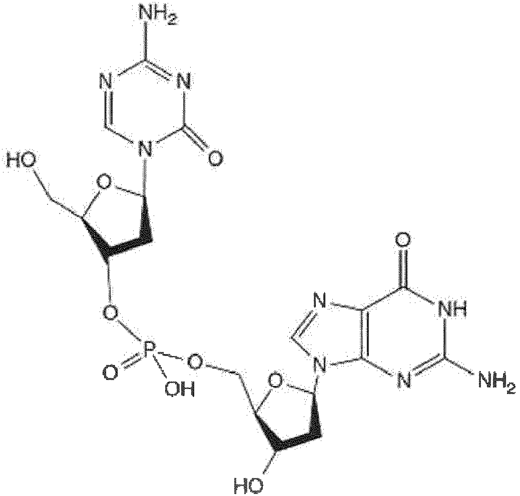 | dinucleotide, prodrug of DAC, improved pharmacokinetics and metabolic stability compared to DAC, in clinical trials for the treatment of leukemia and myelodysplastic syndrome |
| fluorocyclopentenylcytosine [RX-3117] | 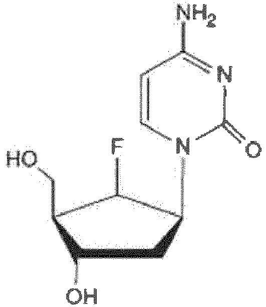 | approved by the FDA for the treatment of pancreatic cancers |
| 2-(p-nitrophenyl) ethoxycarbonyl-5-aza-2'-deoxycytidine [NPEOC-DAC] | 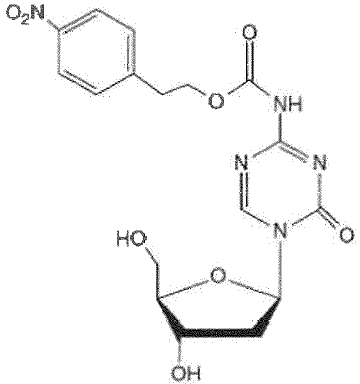 | converted into DAC in cells expressing carboxylesterase 1 |
| CP-4200 | 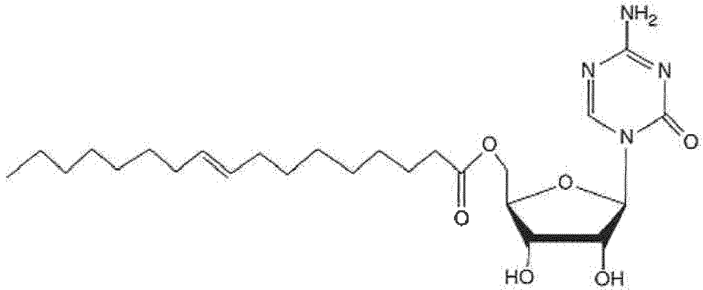 | elaidic acid ester of AZA, prodrug of AZA with delayed delivery of AZA |
| 4'-thio-2'-deoxycytidine [T-dCyd] | 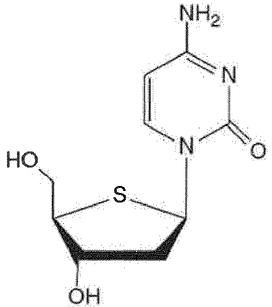 | |
| 5-aza-4'-thio-2'-deoxycytidine [5-aza-T-dCyd] | 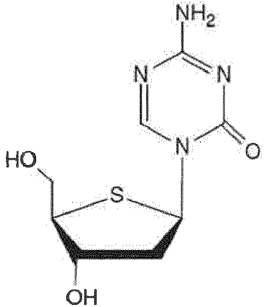 | |
| 1-beta-D-arabinofuranosyl-5-azacytosine [fazarabine] | 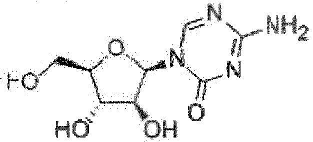 | clinical trials in cancer therapy were stopped (low efficacy) |

In a preferred embodiment, the DNA methyltransferase inhibitor is 5-azacytidine or 5-aza-2'-deoxycytidine. Both 5-azacytidine and 5-aza-2'-deoxycytidine are approved for the treatment of leukemia and myelodysplastic syndrome in Europe, the United States and other countries and are in clinical trials for the treatment of solid tumors.

In another embodiment, the DNA methyltransferase inhibitor is 5-aza-2'-deoxycytidine-p-deoxyguanosine, which is a dinucleotide consisting of 5-aza-2'-deoxycytidine and a deoxygyuanosine. It is an improved version of 5-aza-2'-deoxycytidine with enhanced stability and efficiency and is being tested in clinical trials for leukemia therapy.

In a preferred embodiment, the non-nucleoside compound is selected from the group consisting of
hydralazine,
procaine,
mithramycin A,
nanaomycin A,
N-(4-((2-amino-6-methylpyrimidin-4-yl)amino)phenyl)-4-(quinolin-4-ylamino)benzamide,
*N*-phthalyl-L-tryptophan,
*N*-phthalyl-L-tryptophan derivatives,
alkine derivatives,
halomon,
S-adenosyl-L-methionine analogues,
S-adenosyl-L-homocysteine,
S-adenosyl-L-homocysteine analogues,
MG98,
miR-29a,
miR-29c,
Sinefungin, procainamide,
procainamide derivatives,
procainamide- *N*-phthalyl-L-tryptophan conjugates,
cyclopentathiophene derivatives,
cyclohexathiophene derivatives,
flavone derivatives,
3-nitroflavone derivatives,
flavanones derivatives,
3-chloro-3-nitroflavanone derivatives,
diclone,
laccaic acid,
acridine,
5,5'-Methylenedisalicylic acid,
4-(2-((5-Chloro-2-methoxybenzoyl)amino)ethyl)hydrocinnamic acid,
4-Chloro-N-(4-hydroxy-1-naphthalenyl)-3-nitro-benzenesulfonamide,
(S)-3-(1H-Indol-3-yl)-2-(5-nitro-1,3-dioxo-1,3-dihydro-isoindol-2-yl)-propionic acid,
(R)-2-(1,3-Dioxo-5-phenylethynyl-1,3-dihydro-isoindol-2-yl)-3-(1H-indol-3-yl)-propionic acid,
(S)-2-(2,6-Dioxo-piperidin-1-yl)-3-(1H-indol-3-yl)-propionic acid,
N-hydroxy-4-(2-(4-aminobenzamido)-ethylcarbamoyl)butanamide,
4-amino-N-(2-(ethyl(3-(hydroxyamino)-3-oxopropyl)amino)ethyl)benzamide,
N¹-(2-(4-aminobenzamido)ethyl)-N¹-ethyl-N⁶-hydroxyadipamide,
tetraethylthiuramdisulfide,
(-)-epigallocatechin-3-gallate,
genistein,
curcumin,
psammaplin A, and
psammaplin derivatives.

The chemical structures and examples of the non-nucleoside compounds as well as further information are given in table 2. Alternative names and/or abbreviations of the inhibitors are indicated in square brackets.

**Table 2:**

| **Inhibitor** | **Chemical structure and/or examples** | **Comments and/or reference** |
|---|---|---|
| hydralazine | 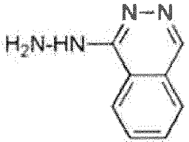 | in clinical trials for cancer therapy |
| procaine | 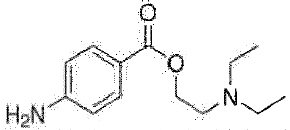 | |
| mithramycin A [MMA] | 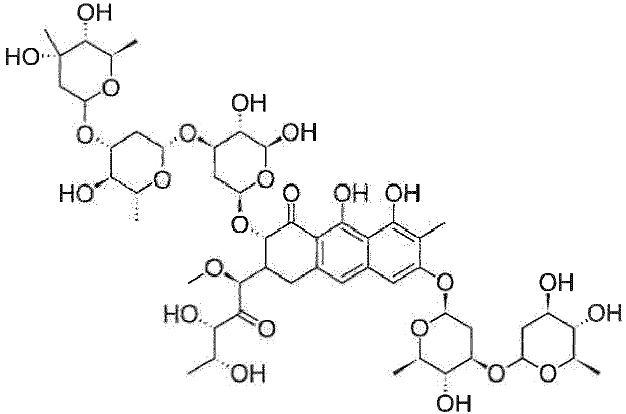 | |
| nanaomycin A | 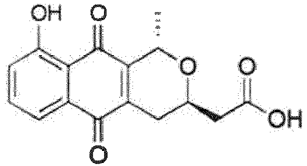 | selective inhibitor of Dnmt3b |
| N-(4-((2-amino-6-methylpyrimidin-4-yl)amino)phenyl)-4-(quinolin-4-ylamino)benzamide [SGI-1027] | 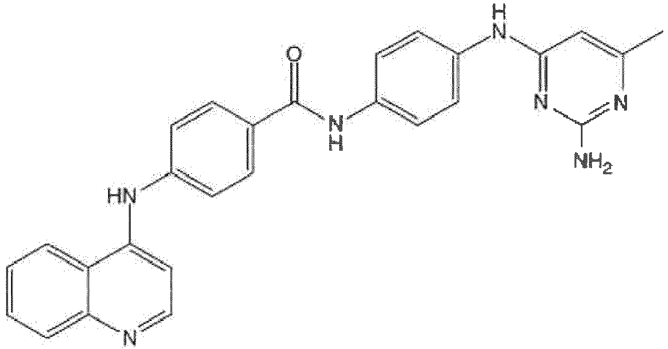 | lipophilic quinoline |
| *N*-phthalyl-L-tryptophan [RG108, phthalimido-L-tryptophan] | 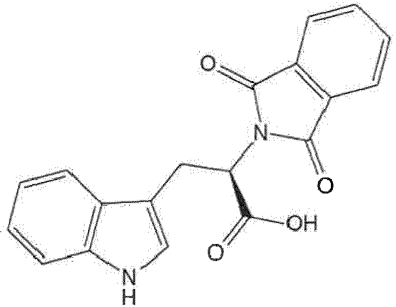 | L-enantiomer of the corresponding racemic NSC401077 |
| *N*-phthalyl-L-tryptophan derivatives [RG108 derivatives] | Examples: | maleimide derivative of RG108 |
| | 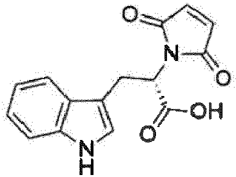 | |
| | 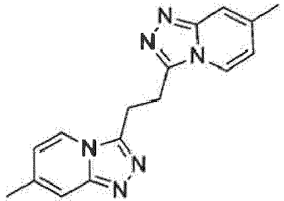 | |
| | 7-methyl-3-[2-(7-methyl-[1,2,4]triazolo[4,3-a]pyridin-3-yl)ethyl]-[1,2,4]triazolo[4,3-a]pyridine [NSC303530] | |
| | 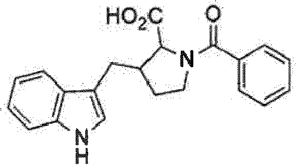 | Pyrrolidino benzoyl analogue |
| | 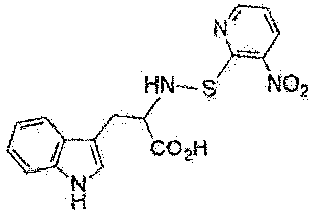 | thionitropyridine analogue |
| alkine derivatives | Example: | WO 2008/098077 A2 |
| | 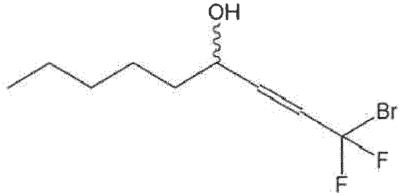 | |
| | 1-Bromo-1,1-difluoro-non-2-yn-4-ol | |
| halomon [(3S,6R)-6-Bromo-3-(bromomethyl)-2,3,7-trichloro-7-methyl-1-octene] | 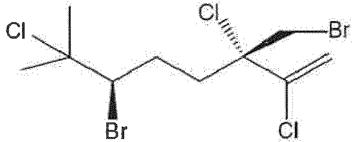 | Polyhalogenated monoterpene |
| S-adenosyl-L-methionine analogues [SAM analogues] | Example: S-adenosyl-L-homocysteine (see below) | |
| S-adenosyl-L-homocysteine [SAH] | 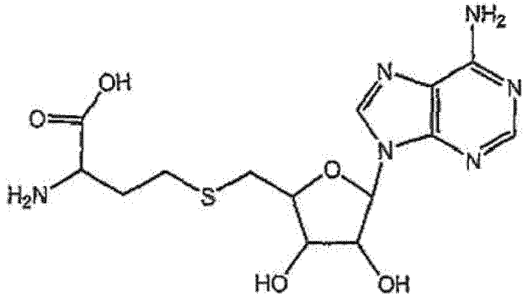 | SAM analogue |
| S-adenosyl-L-homocysteine analogues [SAH analogues] | Examples: | WO 2006/078752 A2 |
| | 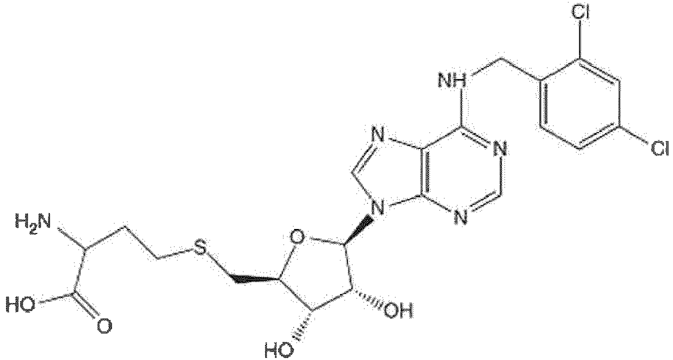 | |
| | 2-amino-4-(((2S,3S,4R,5R)-5-(6-(2,4-dichlorobenzylamino)-9H-purin-9-yl)-3,4-dihydroxytetrahydrofuran-2-yl)methylthio)butanoic acid | |
| | 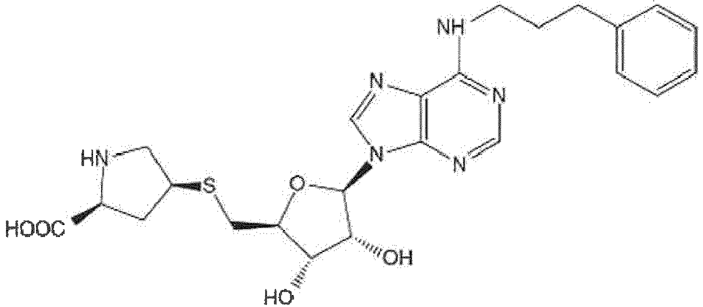 | WO 2006/078752 A2 |
| | (2S,4S)-4-(((2S,3S,4R,5R)-3,4-dihydroxy-5-(6-(3-phenylpropylamino)-9H-purin-9-yl)-tetrahydrofuran-2-yl)methylthio)pyrrolidine-2-carboxylic acid | |
| | 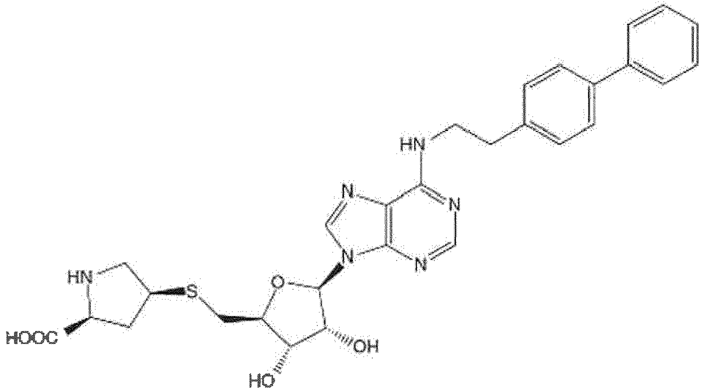 | WO 2006/078752 A2 |
| | (2S,4S)-4-(((2S,3S,4R,5R)-5-(6-(2-(biphenyl-4-yl)ethylamino)-9H-purin-9-yl)-3,4-dihydroxy-tetrahydrofuran-2-yl)methylthio)pyrrolidine-2-carboxylic acid | |
| | 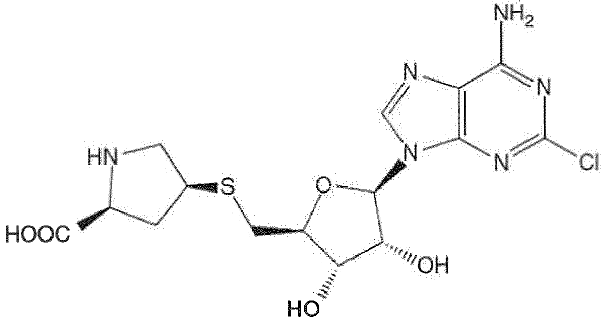 | WO 2006/078752 A2 |
| | (2S,4R)-4-(((2S,3S,4R,5R)-5-(6-amino-2-chloro-9H-purin-9-yl)-3,4-dihydroxy-tetrahydrofuran-2-yl)methylthio)pyrrolidine-2-carboxylic acid | |
| | 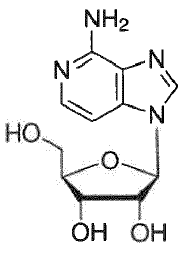 | |
| | 3-deazaadenosine | |
| MG98 | 5'-TTCATGTCAGCCAAGGCCAC-3' (SEQ ID NO.: 1) (nucleotides in bold are 2'-O-methyl modified) | 4X4 hybrid 2'-O-methyl phosphorothioate antisense oligodeoxynu-cleotide directed against DNMT1, does not affect DNMT3, clinical trials in cancer therapy were stopped (low efficacy) |
| miR-29a [hsa-miR-29a-3p] | UAGCACCAUCUGAAAUCGGUUA (SEQ ID NO.: 2) | microRNA targeting Dnmt3a and Dnmt3b |
| miR-29c [hsa-miR-29c-3p] | UAGCACCAUUUGAAAUCGGUUA (SEQ ID NO.: 3) | microRNA targeting Dnmt3a and Dnmt3b |
| Sinefungin | 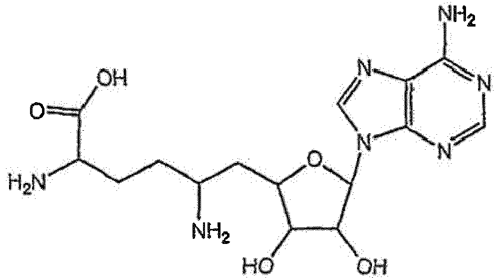 | antifungal antibiotic, SAH analogue |
| procainamide | 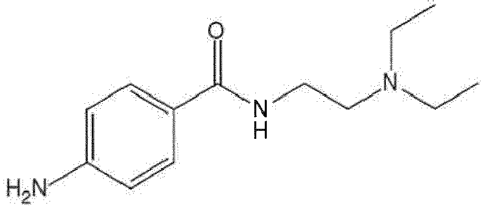 | |
| procainamide derivatives | Examples: | WO 2012/038417 A1 |
| | 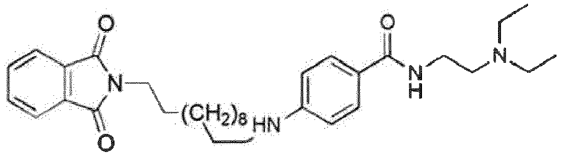 | |
| | N-(2-(diethylamino)ethyl-4-(3-(1,3-dioxoisoindolin-2-yl)dodecylamino)benzamide | |
| | N-(2-(diethylamino)ethyl-4-(3-(1,3-dioxoisoindolin-2-yl)tetradecylamino)benzamide | WO 2012/038417 A1 |
| | 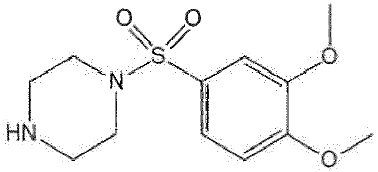 | WO 2012/087889 A2 |
| | 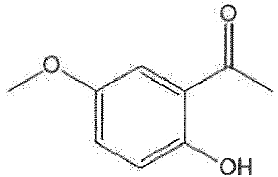 | WO 2012/087889 A2 |
| | 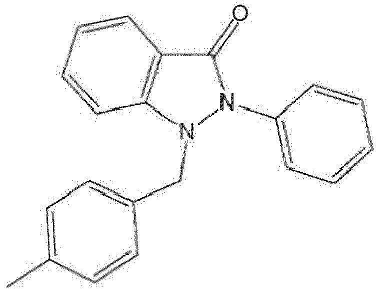 | WO 2012/087889 A2 |
| | 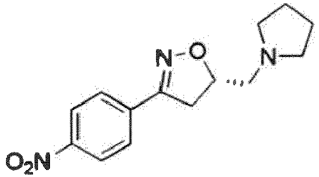 | oxazoline |
| | 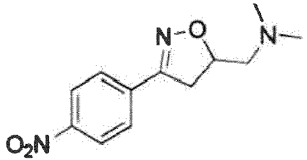 | isoxazoline |
| procainamide-N-phthalyl-L-tryptophan conjugates [procainamide-RG108 conjugates] | Examples: | WO 2012/038417 A1 |
| | 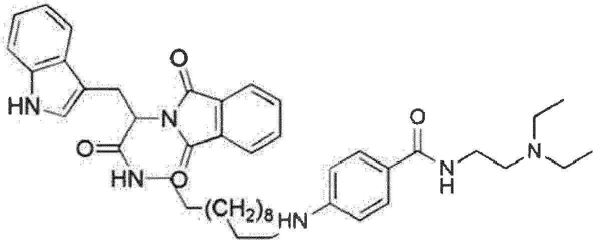 | |
| | N-(2-(diethylamino)ethyl-4-(3-(2-(1,3-dioxoisoindolin-2-yl)-3-(1H-indol-3-yl)propanamido)dodecylamino)benzamide | |
| | N-(2-(diethylamino)ethyl-4-(3-(2-(1,3-dioxoisoindolin-2-yl)-3-(1H-indol-3-yl)propanamido)tetradecylamino)benzamide | WO 2012/038417 A1 |
| cyclopentathiophene derivatives | Example: | WO 2010/098866 A1 |
| | 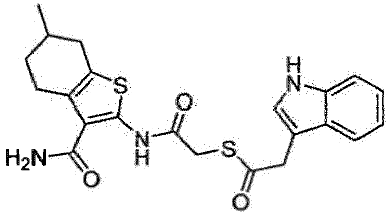 | |
| | S-2-(3-carbamoyl-6-methyl-4,5,6,7-tetrahydrobenzo[b]thiophen-2-ylamino)-2-oxoethyl 2-(1H-indol-3-yl)ethanethioate | |
| cyclohexathiophene derivatives | Example: | WO 2010/098866 A1 |
| | 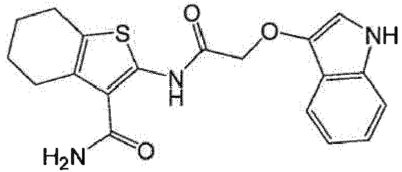 | |
| | 2-(2-(1H-indol-3-yloxy)acetamido)-4,5,6,7-tetrahydrobenzo[b]thiophene-3-carboxamide | |
| flavone derivatives | Example: 3-nitroflavone derivatives (see below) | |
| 3-nitroflavone derivatives | Example: | WO 2011/029956 A1 |
| | 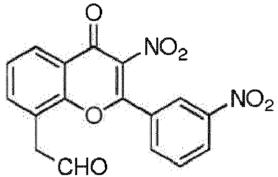 | |
| flavanones derivatives | Example: 3-chloro-3-nitroflavanone derivatives (see below) | |
| 3-chloro-3-nitroflavanone derivatives | Examples: | WO 2011/029956 A1 |
| | 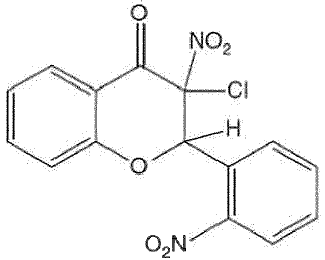 | |
| | 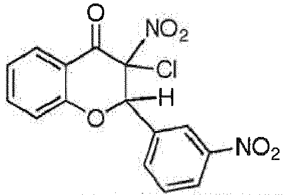 | WO 2011/029956 A1 |
| | 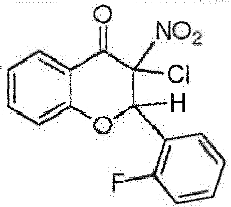 | WO 2011/029956 A1 |
| diclone | 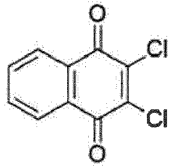 | |
| laccaic acid | 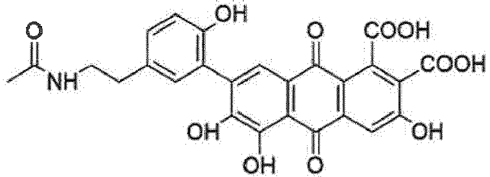 | |
| acridine [Dibenzo[b,e]pyridine] | 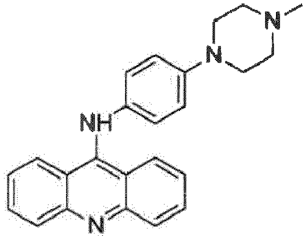 | |
| 5,5'-Methylenedisalicylic acid [NSC] | 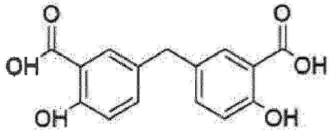 | |
| 4-(2-((5-Chloro-2-methoxybenzoyl)amino)ethyl)hydrocinna mic acid [NSC319745] | 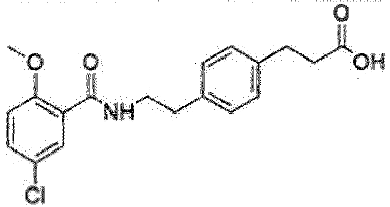 | |
| 4-Chloro-N-(4-hydroxy-1-naphthalenyl)-3-nitrobenzenesulfonamide [SW155246] | 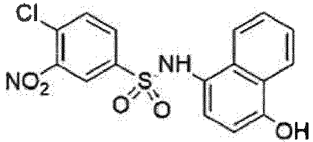 | |
| (S)-3-(1H-Indol-3-yl)-2-(5-nitro-1,3-dioxo-1,3-dihydroisoindol-2-yl)-propionic acid | | WO 2007/007054 A1 |
| (R)-2-(1,3-Dioxo-5-phenylethynyl-1,3-dihydroisoindol-2-yl)-3-(1H-indol-3-yl)-propionic acid | | WO 2007/007054 A1 |
| (S)-2-(2,6-Dioxo-piperidin-1-yl)-3-(1 H-indol-3-yl)-propionic acid | | WO 2007/007054 A1 |
| N-hydroxy-4-(2-(4-aminobenzamido)-ethylcarbamoyl)butanamide | | WO 2008/033744 A2 |
| 4-amino-N-(2-(ethyl(3-(hydroxyamino)-3-oxopropyl)amino)ethyl)benzamide | | WO 2008/033744 A2 |
| N¹-(2-(4-aminobenzamido)ethyl)-N¹-ethyl-N⁶-hydroxyadipamide | | WO 2008/033744 A2 |
| tetraethylthiuramdisulfide [disulfiram] | 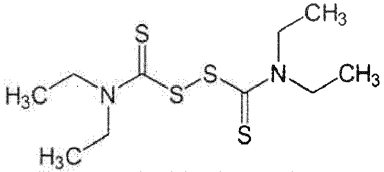 | in clinical trials for cancer therapy |
| (-)-epigallocatechin-3-gallate [EGCG] | 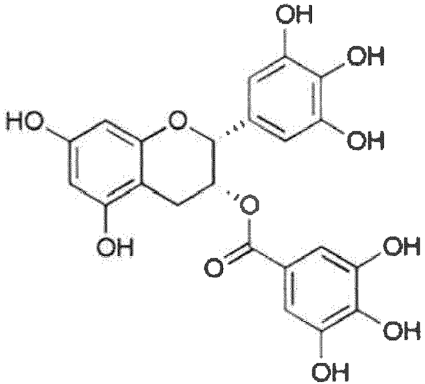 | natural compound (green tea) |
| genistein | 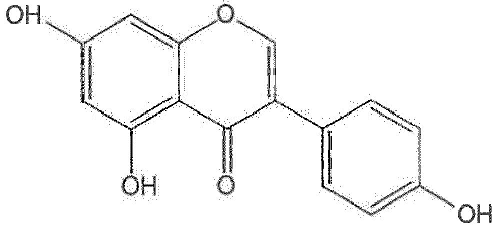 | natural compound (soybean), in clinical trials for the treatment of prostate cancer |
| curcumin | 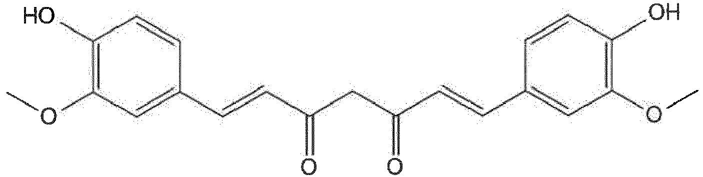 | natural compound (turmeric) |
| psammaplin A | 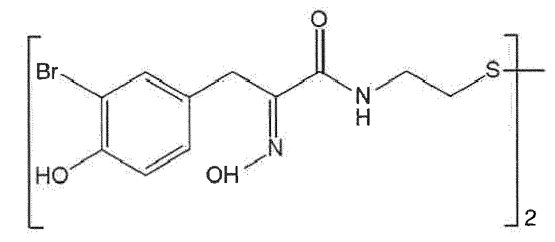 | natural compound (sponge) |
| psammaplin A derivatives | Example: | WO 2008/125988 A1 |
| | 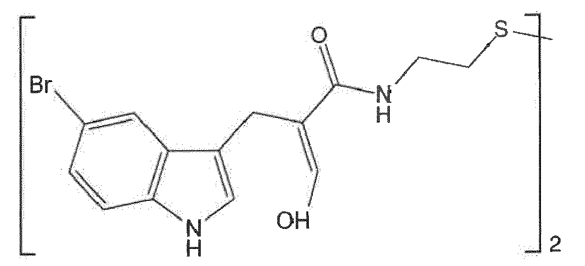 | |

In a preferred embodiment, the DNA methyltransferase inhibitor reduces the enzymatic activity of one or more DNA methyltransferases by at least about 50%, more preferred by at least about 75%, most preferred by at least about 90%.

In addition to Dnmt3a and Dnmt3b, the Dnmt3 family further comprises Dnmt3L. Dnmt3L is homologous to Dnmt3a and Dnmt3b, but lacks enzymatic activity. Dnmt3L has important regulatory functions. For example, binding of Dnmt3L to Dnmt3a activates Dnmt3a and affects its sub-nuclear localization. The inventors found that MeCP2 also interacts with Dnmt3L.

A third catalytically active DNA methyltransferase in mammals is DNA methyltransferase 1 (Dnmt1). Dnmt1 methylates the newly synthesized DNA strand after DNA replication, such that DNA methylation patterns are maintained. It was previously found that MeCP2 interacts with Dnmt1. In contrast to Dnmt3a and Dnmt3b, the enzymatic activity of Dnmt1 is not inhibited by interaction with MeCP2. Nevertheless, a non-specific DNA methyltransferase inhibitor, i.e. an inhibitor that inhibits all DNA methyltransferases, will improve the symptoms of Rett syndrome given that the overall level of DNA methylation is increased compared to healthy individuals.

The use of a non-specific DNA methyltransferase inhibitor may be associated with a higher risk of side effects since it may also cause a decrease in DNA methylation at DNA loci that have had normal methylation levels. A specific DNA methyltransferase 3 inhibitor may act in a more directed fashion and cause a reduction of aberrantly increased DNA methylation only at DNA loci that were directly affected by Dnmt3a and Dnmt3b hyperactivity. Therefore, in a preferred embodiment, the DNA methyltransferase is DNA methyltransferase 3.

In a further preferred embodiment, the DNA methyltransferase 3 is DNA methyltransferase 3a (Dnmt3a). A specific Dnmt3a inhibitor is particularly suited for treating Rett syndrome since Dnmt3a is much more abundant in the brain compared to Dnmt3b.

In a further aspect, the invention relates to a pharmaceutical composition comprising a DNA methyltransferase inhibitor, or a pharmaceutically acceptable salt thereof, and a pharmaceutically acceptable carrier for use in treating Rett syndrome.

The term "pharmaceutically acceptable carrier" as used herein refers to any agent which may be administered to a subject without undue toxicity or side effects in the amounts used. The carrier may be an excipient such as water, saline, dicalcium phosphate, glycerol, sorbitol and/or ethanol. The carrier may be an auxiliary substance such as a wetting agent such as magnesium stearate, an emulsifying agent, a stabilizer and/or a pH buffering substance such as phosphate, citrate, tartrate and/or succinate. The carrier may be a binder such as starch and/or gelatine. Further examples of carriers include mannitol, glucose, lactose, dextrose, xylitol, sucrose, maltose, saccharose, amylopectin, talc, silica, calcium silicate, microcrystalline cellulose, methylcellulose, gum arabicum, vegetable oil, mineral oil, propylene glycol and polyethylene glycol.

The pharmaceutical composition may further comprise thickening agents such as carboxymethylcellulose, sweetening agents such as fructose, flavouring agents such as peppermint and/or preservatives such as methylparaben.

In a preferred embodiment, the DNA methyltransferase inhibitor, or the pharmaceutically acceptable salt thereof, is present in a therapeutically effective amount. The term "therapeutically effective amount" as used herein refers to an amount of the inhibitor that will alleviate or ameliorate one or more of the symptoms of Rett syndrome. The therapeutically effective amount can be determined by conducting clinical studies in subjects having Rett syndrome.

Appropriate dosage of the DNA methyltransferase inhibitor may vary depending on the effectiveness and the pharmacokinetics of the inhibitor as well as on the subject to be treated. For example, age, weight, severity of Rett syndrome symptoms and general condition of the subject may be relevant for choosing the optimal dosage regimen.

In a preferred embodiment, the DNA methyltransferase inhibitor is a nucleoside analogue and/or a non-nucleoside compound.

In a preferred embodiment, the nucleoside analogue is selected from the group consisting of
5-azacytidine,
5-aza-2'-deoxycytidine,
5,6-dihydro-5-azacytidine,
5-fluoro-2'-deoxycytidine,
1-(beta-D-ribofuranosyl)-1,2-dihydropyrimidin-2-one,
5-aza-2'-deoxycytidine-p-deoxyguanosine,
fluorocyclopentenylcytosine,
2-(p-nitrophenyl) ethoxycarbonyl-5-aza-2'-deoxycytidine,
CP-4200,
4'-thio-2'-deoxycytidine,
5-aza-4'-thio-2'-deoxycytidine, and
1-beta-D-arabinofuranosyl-5-azacytosine.

The chemical structures of the nucleoside analogues as well as further information are given in table 1.

In a preferred embodiment, the DNA methyltransferase inhibitor is 5-azacytidine or 5-aza-2'-deoxycytidine. Both 5-azacytidine and 5-aza-2'-deoxycytidine are approved for the treatment of leukemia and myelodysplastic syndrome in Europe, the United States and other countries.

In another embodiment, the DNA methyltransferase inhibitor is 5-aza-2'-deoxycytidine-p-deoxyguanosine, an improved version of 5-aza-2'-deoxycytidine with enhanced stability and efficiency that is being tested in clinical trials for leukemia therapy.

In a preferred embodiment, the non-nucleoside compound is selected from the group consisting of
hydralazine,
procaine,
mithramycin A,
nanaomycin A,
N-(4-((2-amino-6-methylpyrimidin-4-yl)amino)phenyl)-4-(quinolin-4-ylamino)benzamide,
*N*-phthalyl-L-tryptophan,
*N*-phthalyl-L-tryptophan derivatives,
alkine derivatives,
halomon,
S-adenosyl-L-methionine analogues,
S-adenosyl-L-homocysteine,
S-adenosyl-L-homocysteine analogues,
MG98,
miR-29a,
miR-29c,
Sinefungin,
procainamide,
procainamide derivatives,
procainamide- N-phthalyl-L-tryptophan conjugates,
cyclopentathiophene derivatives,
cyclohexathiophene derivatives,
flavone derivatives,
3-nitroflavone derivatives,
flavanones derivatives,
3-chloro-3-nitroflavanone derivatives,
diclone,
laccaic acid,
acridine,
5,5'-Methylenedisalicylic acid,
4-(2-((5-Chloro-2-methoxybenzoyl)amino)ethyl)hydrocinnamic acid,
4-Chloro-N-(4-hydroxy-1-naphthalenyl)-3-nitro-benzenesulfonamide,
(S)-3-(1H-Indol-3-yl)-2-(5-nitro-1,3-dioxo-1,3-dihydro-isoindol-2-yl)-propionic acid,
(R)-2-(1,3-Dioxo-5-phenylethynyl-1,3-dihydro-isoindol-2-yl)-3-(1H-indol-3-yl)-propionic acid,
(S)-2-(2,6-Dioxo-piperidin-1-yl)-3-(1H-indol-3-yl)-propionic acid,
N-hydroxy-4-(2-(4-aminobenzamido)-ethylcarbamoyl)butanamide,
4-amino-N-(2-(ethyl(3-(hydroxyamino)-3-oxopropyl)amino)ethyl)benzamide,
N¹-(2-(4-aminobenzamido)ethyl)-N¹-ethyl-N⁶-hydroxyadipamide,
tetraethylthiuramdisulfide,
(-)-epigallocatechin-3-gallate,
genistein,
curcumin,
psammaplin A, and
psammaplin derivatives.

The chemical structures and examples of the non-nucleoside compounds as well as further information are given in table 2.

In a preferred embodiment, the DNA methyltransferase is DNA methyltransferase 3, preferably DNA methyltransferase 3a. An inhibitor that specifically inhibits Dnmt3 may reduce aberrantly increased DNA methylation only at DNA loci that were directly affected by Dnmt3a and Dnmt3b hyperactivity. It may thus cause less side effects compared to a non-specific DNA methyltransferase inhibitor. A specific Dnmt3a inhibitor is particularly preferred since Dnmt3a is much more abundant in the brain compared to Dnmt3b.

In a further aspect, the invention relates to an *in vitro* method for diagnosing Rett syndrome in a subject comprising the step of analysing DNA methylation in a sample of the subject.

As described above, the inventors found that MeCP2 functions as a negative regulator of global DNA methylation of the genome, primarily reducing overall untargeted DNA methylation of the genome. Since MeCP2 is either dysfunctional or not expressed in Rett syndrome patients, the repression of the enzymatic activity of Dnmt3a and Dnmt3b is missing. As a result, Dnmt3a and Dnmt3b are hyperactive and the overall level of DNA methylation of the genome is increased compared to healthy individuals. Therefore, the analysis of DNA methylation in a sample of a subject suspected of having Rett syndrome allows diagnosing Rett syndrome.

The term "subject" as used herein refers to a mammal. The mammal is preferably a human or a mouse, most preferred a human.

In a preferred embodiment, analysing the DNA methylation comprises the steps of quantifying the level of DNA methylation in the sample; and comparing the level of DNA methylation with a reference value obtained from a reference sample of a healthy individual, wherein an increased level of DNA methylation in the sample compared to the reference value indicates that the subject has Rett syndrome.

DNA methylation is naturally present in healthy individuals. Thus, for the interpretation of test results and obtaining a diagnosis, the quantity of DNA methylation detected within the sample of the subject is preferably compared to a standardized reference value. The reference value is the level of DNA methylation obtained from at least one reference sample of at least one healthy individual. Due to a certain variability of the level of DNA methylation within the healthy population, the reference value is preferably determined by a comprehensive survey of the healthy population.

The finding that the level of DNA methylation is increased in subjects having Rett syndrome compared to healthy individuals facilitates the diagnosis of Rett syndrome by comparing the level of DNA methylation in the sample with a reference value obtained from a reference sample of a healthy individual.

To limit variations that may result from different analytical techniques, it is preferred to apply the same technique for analysing DNA methylation in the sample and in the reference sample.

Methods for analysing DNA methylation are well established in the art. The detection of 5-methylcytosine is commonly achieved by DNA modification by bisulfite conversion, a method also known as bisulfite sequencing. Bisulfite conversion facilitates the identification and quantification of DNA methylation at single nucleotide resolution. A detailed protocol can be found for example in Patterson et al. 2011. In short, the genomic DNA first needs to be isolated from the sample and denatured. It is then treated with sodium bisulfite so that an addition of bisulfite to the 5-6 double bond of cytosine takes place. This step is followed by hydrolytic deamination of the cytosine-bisulfite derivative, whereby a uracil-bisulfite derivative is obtained. The sulfonate group of the uracil-bisulfite derivative is then removed by an alkali treatment which results in uracil. Bisulfite preferentially deaminates cytosine to uracil in single stranded DNA. In contrast, 5-methylcytosine is not affected by bisulfite treatment. Therefore, upon PCR amplification, uracil is amplified as thymine while 5-methylcytosine residues remain as cytosines. Thus, methylated CpGs can be distinguished from unmethylated CpGs by the presence of a cytosine versus a thymine during sequencing of the PCR products.

Bisulfite conversion may be combined with an enzymatic restriction digest using a methylation sensitive endonuclease. Such a combined bisulfite restriction analysis (COBRA) may be further combined with high-throughput genome sequencing, thereby facilitating DNA methylation analysis at single base pair resolution.

Bisulfite conversion may be followed by methylation-specific PCR (MSP) using primers specific for either methylated or unmethylated DNA based on the sequence differences resulting from bisulfite treatment. This approach requires only small amounts of DNA.

MSP and further PCR-based methods for analysing DNA methylation are described for example in Hernández et al. 2013.

The level of DNA methylation can also be quantified by bisulfite-free methods such as digesting the DNA by a methylation sensitive restriction endonuclease and amplifying the digested DNA by PCR using primers that span the restriction site of the endonuclease. Due to the digest prior to PCR, only methylated DNA templates will be amplified.

Another bisulfite-free method for analysing DNA methylation is methylated DNA immunoprecipitation (MeDIP) in which methylated DNA is bound to 5-methylcytosine-specific (anti-5mC) antibodies and isolated with microparticles (beads) conjugated to secondary antibodies that bind the anti-5mC antibodies used.

DNA methylation can further be analysed using methyl-CpG-binding proteins or methyl-CpG-binding protein domains such as MBD2 which bind to doublestranded CpG-methylated DNA with high affinity. One of the assays using this approach is methylated CpG island recovery assay (MIRA). Methylated DNA that is bound to the protein or protein domain can be isolated by precipitation, for example when GST-tagged MBD2 and glutathione beads are used. MIRA can also be combined with microarray platforms, facilitating the analysis of DNA methylation on a genome-wide scale.

In a preferred embodiment, the sample comprises brain cells, preferably neurons. The MeCP2 protein is highly expressed in neurons and has important functions in the brain. Therefore, the effect of dysfunctional or absent MeCP2 protein on the level of DNA methylation can be particularly well observed in brain cells, especially in neurons.

In a further aspect, the invention relates to a kit for diagnosing Rett syndrome in a subject *in vitro* comprising reagents for quantifying a level of DNA methylation in a sample of the subject, wherein an increased level of DNA methylation in the sample compared to a reference value obtained from a reference sample of a healthy individual indicates that the subject has Rett syndrome.

As described above, increased DNA methylation in subjects having Rett syndrome compared to healthy individuals allows diagnosing Rett syndrome by comparing the level of DNA methylation in the sample of the subject with the reference value obtained from at least one reference sample of at least one healthy individual.

The reagents for quantifying the level of DNA methylation may comprise bisulfite, reagents for alkali treatment for removing the sulfonate group of the uracil-bisulfite derivative such as sodium hydroxide, reagents for PCR amplification and/or a methylation sensitive endonuclease such as *Taql*.

In a preferred embodiment, the reagents for quantifying the level of DNA methylation comprise bisulfite. The analysis of DNA methylation by bisulfite treatment is a simple and well established procedure.

In another embodiment, the reagents for quantifying the level of DNA methylation comprise anti-5mC antibodies such as mouse anti-5mC antibodies. In this case, the reagents preferably further comprise beads conjugated to anti-mouse antibodies. These reagents facilitate quantifying the level of DNA methylation by MeDIP.

In another embodiment, the reagents for quantifying the level of DNA methylation comprise a methyl-CpG-binding protein or methyl-CpG-binding protein domain such as MBD2 so that the level of DNA methylation can be analysed on doublestranded DNA.

The kit may further comprise a lysis buffer for isolating DNA from the sample and/or reagents for denaturing the DNA such as sodium hydroxide.

In a preferred embodiment, the sample comprises brain cells, preferably neurons. As described above, the MeCP2 protein is highly expressed in neurons and has important functions in the brain. Therefore, the effect of inactive or absent MeCP2 protein on the level of DNA methylation can be particularly well observed in brain cells, especially in neurons.

Further disclosed is a method of treating Rett syndrome comprising administering a therapeutically effective amount of a DNA methyltransferase inhibitor or a pharmaceutically acceptable salt thereof.

The term "therapeutically effective amount" refers to an amount of the inhibitor that will alleviate or ameliorate one or more of the symptoms of Rett syndrome.

For human and mouse Dnmt and MeCP2 proteins used herein, the Uniprot entry name, the Uniprot accession number, the Uniprot entry version and the Uniprot identifier are given in table 3. The information is taken from the Uniprot (Universal Protein Resource) database.

**Table 3:**

| Protein | Uniprot entry name | Uniprot accession number | Uniprot entry version | Uniprot identifier |
|---|---|---|---|---|
| human Dnmt1 | DNMT1_HUMAN | P26358 | 182 | P26358-1 |
| mouse Dnmt1 | DNMT1_MOUSE | P13864 | 183 | P13864-1 |
| human Dnmt3a | DNM3A_HUMAN | Q9Y6K1 | 146 | Q9Y6K1-1 |
| human Dnmt3a2 | DNM3A_HUMAN | Q9Y6K1 | 146 | Q9Y6K1-2 |
| mouse Dnmt3a | DNM3A_MOUSE | 088508 | 162 | 088508-1 |
| mouse Dnmt3a2 | DNM3A_MOUSE | 088508 | 162 | 088508-2 |
| human Dnmt3b | DNM3B_HUMAN | Q9UBC3 | 160 | Q9UBC3-1 |
| human Dnmt3b2 | DNM3B_HUMAN | Q9UBC3 | 160 | Q9UBC3-2 |
| mouse Dnmt3b | DNM3B_MOUSE | 088509 | 145 | 088509-1 |
| mouse Dnmt3b2 | DNM3B_MOUSE | 088509 | 145 | 088509-2 |
| human Dnmt3L | DNM3L_HUMAN | Q9UJW3 | 133 | Q9UJW3-1 |
| mouse Dnmt3L | DNM3L_MOUSE | Q9CWR8 | 119 | Q9CWR8-1 |
| human MeCP2 | MECP2_HUMAN | P51608 | 193 | P51608-1 |
| mouse MeCP2 | MECP2_MOUSE | Q9Z2D6 | 152 | Q9Z2D6-1 |

### Examples

### Materials and methods

### Generation of Dnmt3a2 mutants and MeCP2 domain constructs

The Dnmt3a and MeCP2 proteins and protein domains were prepared as indicated in Figure 1. The MeCP2 domains and mutants proteins were cloned in pGEX-6P2 vector using BamHI and Xhol cloning sites. All constructs were verified by DNA sequencing. The site directed mutagenesis were carried out by rolling circle PCR using a primer carrying point mutation (Jeltsch and Lanio, 2002). The presence of the mutations was confirmed by restriction marker analysis and by DNA sequencing.

### Expression and purification of MeCP2 proteins and Dnmt3a proteins

The MeCP2 domains and mutants proteins were expressed in *E*. *coli* BL-21 cells. Cells were cultivated in LB medium at 37 °C while shaking until an OD(600nm) of 0.6 - 0.7 was reached. Then, protein expression was induced by addition of 1 mM of isopropyl-β-D-thiogalactoside (IPTG) and the culture was incubated at 18 °C shaking at 200 rpm overnight. The cells were harvested by centrifugation (15 min at 4600 rpm) and the pellet resuspended in sonication buffer (20 mM HEPES pH 7.5, 500 mM KCI, 1 mM EDTA, 1 mM DTT, 10% glycerol) including protease inhibitor (Sigma). The cells were lysed by sonication and centrifuged at 18000 rpm for 1 h to prepare a clear lysate, which was applied on a GST-sepharose column (GE Healthcare). After washing with sonication buffer, the protein was eluted with sonication buffer containing 50 mM reduced glutathione and dialyzed first against dialysis buffer I (20 mM HEPES pH 7.5, 200 mM KCI, 1 mM EDTA, 1 mM DTT, 10% glycerol) for three hours, then against dialysis buffer II containing 60% glycerol overnight. The purified protein was analyzed on 12% SDS-PAGE gel stained with colloidal Coomassie Brilliant Blue. The murine Dnmt3a2 and Dnmt3a-C proteins were expressed and purified as described in Jia et al. 2007. Since all Dnmt3a structures were annotated with numbers for the human proteins, human numbering is used here. The residue numbers corresponding to human D528, D531 and Q527 in murine Dnmt3a are D524, D527 and Q523, respectively.

### GST pull-down experiments

For GST pull-down experiments 20 µL of Glutathione-Sepharose 4B beads were washed with 200 µL of interaction buffer (25 mM Tris pH 8.0, 100 mM KCI, 5 mM MgCl2, 10% glycerol, 0.1 % NP40, 200 µM PMSF). The beads were incubated for 1 h at 4 °C with 10-15 µg of the different GST-tagged proteins, washed three times with interaction buffer and incubated with His- or MBD-tagged proteins (15 µg) for 1 hour at 4 °C with shaking. Then, the beads were washed three times with wash buffer containing high salt (25 mM Tris pH 8.0, 5 mM MgCl2, 300 mM KCI, 10 % glycerol, 0.1 % NP40, 200 µM PMSF). The interaction of Dnmt3a ADD and MeCP2 TRD domains was also investigated using buffer containing 600 mM KCI. Finally, the beads were resuspended in SDS gel loading buffer and incubated for 10 min at 95 °C. After centrifugation of the beads at 14,000 rpm for 10 min the supernatant was loaded on a 12% SDS PAGE gel. Proteins were detected by Western blotting or Coomassie Brilliant Blue staining as indicated. Some experiments were conducted in the presence of recombinant Histone H3.1 (M2503S, NEB), as detailed in the results section.

### Co-immunoprecipitation assay

For co-immunoprecipitation of Dnmt3a and MeCP2, pcDNA DNMT3A (expressing myc-tagged Dnmt3a) and pEYFP-MeCP2 plasmids were co-transfected in HEK293 cells. The pEYFP plasmid was used as control. After 48 h, the cells were collected and the cell pellets stored at -80 °C. The cells were lysed as recommended by the GFP trap protocol (ChromTek). Using GFP trap YFP-tagged MeCP2 was pulled-down and the complex washed with buffer (10 mM Tris/Cl pH 7.5, 0.5 mM EDTA, 0.5% NP-40, 200 mM NaCl). The MeCP2 and Dnmt3a proteins were separated on a 12% SDS-PAGE and transferred to nitrocellulose membrane. To detect Dnmt3a, the blot was probed with anti-myc antibody (Santa Cruz, 1:1000 dilution) for 1 h at room temperature.

For immunoprecipitation of endogenous Dnmt3a and MeCP2, whole brains from sixteen-week old C57BI/N female mice were used. Following mechanical disruption, the tissue was lysed. Three brains were homogenized in NP-40 lysis buffer (10 mM HEPES, pH 7.9, 3 mM MgCl₂, 10 mM KCI, 10 mM NaF, 1 mM Na₃VO₄, 0.5 mM DTT, 0.5% NP-40, 1x complete EDTA-free protease inhibitor cocktail (Roche)) by douncing 30 times with a tight pestle, and pelleted at 1,000 g. Lysates were next diluted 1:1 with Benzonase buffer (10 mM HEPES, pH7.9, 3 mM MgCl₂, 280 mM NaCl, 0.2 mM EDTA, 10 mM NaF, 1 mM Na₃VO₄, 0.5 mM DTT, 0.5% NP-40) supplemented with 1x complete EDTA-free protease inhibitor cocktail (Roche) and sonicated with EpiShear (Active Motif) for 2 min 30 sec (15 sec ON, 30 sec OFF cycles, 20% power, 3.2 mm microtip). The homogenate was then digested with 500 units of Benzonase (Novagen) for 2 h rotating at 4 °C. Chromatin proteins were separated by centrifugation at 17.000 g for 20 min at 4 °C. For each pull-down, 2.5 mg lysate were pre-cleared for 1 h at 4 °C with 20 mg Protein A Sepharose CL-4B (GE Healthcare), followed by overnight incubation with 15 µg anti-Dnmt3a antibody (sc-2070, Santa Cruz). For negative control, an equivalent amount of non-related rabbit IgG anti-myc (ab9106, Abcam) antibody was used. The antibody-bound proteins were immobilized to 100 mg Protein A Sepharose CL-4B, blocked in 10% BSA (Roth) for 6 h rotating at 4 °C. After five washes with immunoprecipitation buffer, the immune complexes were eluted from the beads by boiling in 100 µL Laemmli sample buffer. The samples were next analyzed by western blot as described above. For detection, anti-MeCP2 monoclonal primary antibody (#3456, Cell Signaling) was used, followed by incubation with HRP-linked anti-rabbit IgG light chain specific secondary antibody (211-032-171, Jackson ImmunoResearch). Western lighting *Ultra* (Perkin Elmer) was used as ECL HRP substrate.

### Fluorescence microscopy

For localization studies, NIH3T3 cells were seeded on glass slides and transfected with plasmids expressing CFP- and YFP-tagged Dnmt3L, Dnmt3a-ADD and MeCP2 using Fugene HD (Promega) according to the manufacturer's instructions. After 48 h, the cells were fixed with 4% formaldehyde, mounted in Mowiol and Z stacks images were collected using a Zeiss LSM 710 confocal microscope.

### Substrates used for DNA methylation

The following oligonucleotide substrates were used for DNA methylation assays: a biotinylated unmethylated 30mer containing one CpG site (um30mer; upper strand: TTG CAC TCT CCT CCC GGA AGT CCC AGC TTC (SEQ ID NO.: 4); lower strand, 5'-biotinylated: GAA GCT GGG ACT TCC GGG AGG AGA GTG CAA (SEQ ID NO.: 5)), the same sequence hemimethylated at the CpG site with the methylation in the lower DNA strand (hm30mer; upper strand: TTG CAC TCT CCT CCC GGA AGT CCC AGC TTC (SEQ ID NO.: 6); lower strand, 5'-biotinylated: GAA GCT GGG ACT TC^{m}C GGG AGG AGA GTG CAA (SEQ ID NO.: 7)), a biotinylated hemimethylated 30mer with optimized flanks for methylation with Dnmt3a (hmF30mer; upper strand: GAA GCT GGA CAG TAC GTC AAG AGA GTG CAA (SEQ ID NO.: 8); lower strand, 5'-biotinylated: TTG CAC TCT CTT GA^{m}C GTA CTG TCC AGC TTC (SEQ ID NO.: 9)) and a non-CpG substrate (nonCpG; upper strand: GAA GCT GGT CCA TT^{m}C GAT GAT GGA GTG CAA (SEQ ID NO.: 10); lower strand, 5'-biotinylated: TTG CAC TCC ATC AT^{m}C GAA TGG ACC AGC TTC (SEQ ID NO.: 11)). The oligonucleotides were annealed by heating to 86 °C for 5 min and slowly cooling down to ambient temperature. In addition, a biotinylated 585mer DNA substrate obtained by PCR was used which contains 8 Hpall sites (CCGG) and 45 CpG sites (um585mer). The 585mer was amplified from lambda DNA by PCR using the following primers: forward primer, 5'-biotinylated: GAA GGA CAA CCT GAA GTC CAG GTTG (SEQ ID NO.: 12); and reverse primer: GTG TAT GAC CAC CAG AGC CTT TTGC (SEQ ID NO.: 13). The 585mer was purified by PCR purification kits (Qiagen). To prepare partially methylated 585mer (pm585mer), the DNA was methylated with M.Hpall (NEB) following the protocol of the provider and afterwards purified by PCR purification kits. Successful pre-methylation at Hpall sites was confirmed by Hpall (NEB) restriction digestion.

### DNA methylation activity assay

The avidin-biotin microplate Dnmt activity assay was used to monitor the activity of different Dnmt3a variants in the methylation of biotinylated DNA substrates. Each well of the microplate was coated with 1 µg of Avidin dissolved in 100 µL of 100 mM NaHCO₃ (pH 9.6) and incubated overnight at 4°C. Before starting the assay, the wells were washed five times with 200 µL of 1xPBST (140 mM NaCl, 2.7 mM KCI, 4.3 mM Na₂HPO₄, 1.4 mM K₂HPO₄, 0.05% v/v Tween 50, pH 7.2) containing 0.5 M NaCl. The reaction mixtures were prepared containing 2.5 µM Dnmt3a2 or Dnmt3a-C and 3 µM MeCP2 (or any of its domains such as its TRD domain) in methylation buffer (20 mM HEPES pH 7.2, 1 mM EDTA, 50 mM KCI, 1.25 mg/ml bovine serum albumin). For the control reactions without MeCP2, the same volume of dialysis buffer II was added instead of the MeCP2. The reaction mixtures were incubated on ice for 20 minutes and the wells of the plate were filled with 5 µL of 0.5 M unlabeled AdoMet (Sigma) dispensed in 35 µL 1x PBST/0.5 M NaCl. Then, 1 µM 30mer oligonucleotide DNA or 100 nM of 585mer DNA and 0.76 µM [methyl-3H]-AdoMet (PerkinElmer Life Sciences) were added to the reaction mixture and the samples incubated at 37°C. In order to follow the time course of the reaction, aliquots of 2 µL were removed from the reaction mixtures in duplicates at time points between 2 minutes and 30 minutes and applied to one well of the microplate where the incorporation of labeled AdoMet was quenched by an excess of unlabeled AdoMet. This mixture was incubated while slightly shaking for 30 minutes to 1 hour. 200 µL of 1 x PBST and 0.5 M NaCl were used to wash the wells five times. 40 µL of unspecific nuclease from *Serratia marcescens* was added, diluted 1:2500 in 50 mM Tris/HCl pH 8.0, 5 mM MgCl₂ were added per well and the mixture was incubated for 30 to 60 minutes with slight shaking. Finally, the released radioactivity was measured using liquid scintillation counting and the average count per minute of the duplicates was plotted against time. Linear regression was used to obtain the slopes of the initial linear parts of the time courses. The data are reported as averages and SEM (standard error of the mean) of at least two independent experiments.
The same assay was used to study the DNA methylation activity of Dnmt3b2, an artificially created Dnmt3b variant that mimics the domain structure of Dnmt3a2.

### DNA methylation analysis in HCT116 DNMT1 hypomorphic cells

To study the effect of MeCP2 on Dnmt3a mediated methylation in cells, the inventors used HCT116 DNMT1 hypomorphic cells, which have a reduced DNA methylation (Egger et al. 2006). HCT116 cells were cultivated in McCoy's 5A medium (Gibco) supplemented with 10% heat-inactivated calf serum and 2 mM L-glutamine (Sigma), at 37°C in a saturated humidity atmosphere containing 5% CO₂. For stable cell line generation, pEYFP-NLS or pEYFP-MeCP2 plasmids were transfected into HCT116 cells using jetPRIME (Peqlab), following standard protocols. At 24 hours post-transfection the cells were subjected to selection with 0.5 mg/mL G418 (Sigma-Aldrich). For the pEYFP-MeCP2-transfected cells, stable clonal populations were derived by clonal expansion of single cell dilutions. For pEYFP-NLS, a mixed clonal population was derived. The cultures, together with untransfected control cells, were passaged for 2 months before genomic DNA isolation. LC-ESI-MS/MS analysis of DNA methylation in genomic DNA was performed as described in Bashtrykov et al. 2014.

### Results

### Dnmt3 proteins interact with MeCP2

To test whether Dnmt3a and MeCP2 interact, GST-pull-down assays using recombinant full-length (FL) proteins (Figure 1) were performed. MeCP2 could be obtained with good purity after generating a version that lacked the N-terminal unstructured domain (MeCP2ΔN, amino acid residues 104-486). MeCP2 carries an endogenous His-tag. As shown in Figure 2A, a robust pull-down of Dnmt3a by GST-MeCP2ΔN could be detected. Similar experiments demonstrated the interaction of GST-MeCP2ΔN with Dnmt3a2 (Figure 2B), a shorter isoform of Dnmt3a that is predominantly expressed in embryonic stem cells. Furthermore, MeCP2ΔN was shown to pull-down Dnmt3L, a non-catalytic member of the Dnmt3 family with important regulatory functions (Figure 2C). However, no interaction of MeCP2ΔN with the C-terminal domain of Dnmt3L (Dnmt3L-C) could be detected (Figure 2D). Pull-down experiments with Dnmt3a-C failed, because this protein showed unspecific binding to the GST beads. It was further tested whether the interaction between Dnmt3a and MeCP2 also occurs in cells by transient co-expression of Myc-tagged Dnmt3a and EYFP-tagged MeCP2 in HEK293 cells, followed by EYFP tar-geted purification. As shown in Figure 2E, co-purified Myc-Dnmt3a was detected after co-expression with EYFP-MeCP2, but not with the EYFP control. To exclude potential artifacts related to protein over-expression, the inventors finally performed immuno-precipitation of endogenous Dnmt3a from mouse brain protein extracts. As shown in Figure 2F, the presence of MeCP2 could be specifically detected in the pulled-down material, but not in the control. Together, these results demonstrate that Dnmt3a and MeCP2 interact *in vitro,* after transient expression in cells and with endogenous proteins in brain extracts.

### The interaction between MeCP2 and Dnmt3 proteins is mediated by their TRD and ADD domains

The fact that MeCP2 interacts with the full-length Dnmt3 proteins, but not with Dnmt3L-C suggests that MeCP2 binds to the ADD domain of Dnmt3a and Dnmt3L. To test this hypothesis, pull-down assays were performed with GST-tagged N-terminal domains of Dnmt3a as bait and GST-cleaved MeCP2ΔN as prey. As documented in Figure 3A, MeCP2ΔN showed a specific interaction with the ADD domain of Dnmt3a, but not with its N-terminal domain (NTD) or PWWP domains. To dissect which part of MeCP2 is responsible for the interaction with Dnmt3a, pull-down assays with GST-tagged fused MeCP2 domains were performed. As shown in Figure 3B, interaction was detectable only for the TRD domain. The MeCP2 TRD domain also showed interaction with Dnmt3a2 (Figure 3C). This interaction was stable even in the presence of high salt conditions (600 mM KCI). Pull-down assays using the isolated MeCP2-TRD and Dnmt3a-ADD domains confirmed the direct and strong interaction (Figure 3D). Since MeCP2 is known to be mutated in Rett syndrome, the inventors also tested the effect of the R306C Rett mutation in the TRD domain of MeCP2 on the interaction with Dnmt3a, but did not observe any changes when compared with the wild type TRD domain (Figure 3D).

### The interaction with MeCP2 inhibits the catalytic activity of Dnmt3a

To elucidate the function of the interaction between MeCP2 and Dnmt3a, the inventors measured *in vitro* rates of DNA methylation by Dnmt3a in the presence of MeCP2 or its TRD domain with 5 different DNA substrates, viz. an unmethylated 30mer oligonucleotide (um30mer), the same sequence in a hemimethylated state (hm30mer), a hemimethylated 30mer with an optimized flank for Dnmt3a (hmF30mer), and a 585mer PCR fragment (um585mer) (Figure 4A). Using 2.5 µM Dnmt3a2 and 3 µM MeCP2, it was consistently observed that the interaction of MeCP2 with Dnmt3a2 resulted in about 40-60% reduction in enzymatic activity with the unmethylated substrates. The activity of Dnmt3a2 was further reduced by about 80% with methylated substrates, which can be attributed to a better binding of MeCP2 to methylated DNA via its methyl-CpG-binding domain (MBD domain). The inventors speculated that binding of MeCP2 to pre-methylated DNA might target Dnmt3a2. To test this hypothesis, a partially methylated 585mer DNA substrate was prepared by methylation of the DNA substrates with Hpall methyltransferase, which methylates all CCGG sites. However, even on this substrate, inhibition of the activity of Dnmt3a2 by MeCP2 was observed (Figure 4A, pm585mer).

As a control, Dnmt3a-C, which lacks the ADD domain and does not interact with MeCP2, was used and it was observed that its activity was not inhibited or only very weakly reduced by MeCP2 (Figure 4B). It was further tested if the isolated MeCP2 TRD domain also inhibits the activity of Dnmt3a2 and 40-70% of inhibition with the different substrates was observed (Figure 4C). The inventors have previously discovered that Dnmt3a also methylates non-CpG sites, preferably in a CA context. Non-CpG methylation has been detected in considerable amounts in human embryonic stem cells and neurons and it was shown that this modification is connected with Dnmt3a activity (e.g. Guo et al. 2014). Therefore, the inventors also investigated the influence of MeCP2 on the non-CpG methylation activity of Dnmt3a2 using a 30mer oligonucleotide substrate which contains one already methylated CpG site, such that additional methylation could only occur at non-CpG sites. As with the other DNA substrates, a similar inhibition of Dnmt3a2 activity by the TRD domain was observed (Figure 4C, non-CpG 30mer), indicating that CpG and non-CpG methylation are inhibited to a similar extent by the TRD interaction.

The inventors further tested the inhibition of Dnmt3a2 by increasing amounts of the MeCP2 TRD domain and observed that the methyltransferase activity is strongly inhibited (>95%) using a 2.4 fold excess of TRD (6 µM with 2.5 µM Dnmt3a2) (Figure 4D). As a control, the same experiments were conducted with Dnmt3a-C, but here only a very weak reduction of activity was observed (Figure 4D). This result indicates that the inhibition of Dnmt3a2 by the TRD domain is not caused by competition for the DNA substrate, which is an important control, since the TRD domain has been reported to show DNA binding. In summary, the results indicate that the interaction between the TRD domain of MeCP2 and the ADD domain of Dnmt3a results in a direct and very strong inhibition of the DNA methylation activity of Dnmt3a2 on CpG and non-CpG sites.

### The interaction with the MeCP2 TRD domain also inhibits the catalytic activity of Dnmt3b

It is known that the ADD domains as well as all the residues that are involved in the intramolecular interaction between the ADD domain and the catalytic domain are highly conserved between Dnmt3a and Dnmt3b (Guo et al. 2015). Thus, the inventors also tested the inhibition of Dnmt3b2 (an artificially created Dnmt3b variant that mimics the domain structure of Dnmt3a2) by the MeCP2 TRD domain and observed that the presence of the TRD domain resulted in about 40% reduction in methyltransferase activity of Dnmt3b2 using the unmethylated um30mer substrate (Figure 5).

### The interaction with MeCP2 leads to changes in the sub-nuclear localization of the Dnmt3a ADD domain and Dnmt3L

To study the cellular effect of the interaction of Dnmt3 proteins with MeCP2, the inventors took a closer look at the localization of both proteins and protein domains. Dnmt3a and MeCP2 were both known to localize to DAPI-stained heterochromatin, which forms characteristic spots in mouse NIH3T3 cells. This natural co-localization precluded direct studies of the mutual effect of both proteins on their sub-nuclear localization. However, Dnmt3L was shown to have an almost homogenous nuclear distribution. Transient expression of both Dnmt3L and MeCP2 with fluorescence tags in NIH3T3 cells confirmed the known localization patterns. Interestingly, co-expression of Dnmt3L with MeCP2 led to a clear redistribution of Dnmt3L towards the heterochromatic spots. This finding confirms the intracellular interaction of Dnmt3L and MeCP2 and indicates that interaction with MeCP2 changes the sub-nuclear localization of Dnmt3L. The inventors also performed localization studies with the fluorescently tagged Dnmt3a-ADD domain, which showed a homogenous nuclear distribution. Similarly to Dnmt3L, after co-expression with CFP-tagged MeCP2, the ADD domain showed a preferential localization to heterochromatic spots, indicating that MeCP2 targets it to heterochromatin. These results confirm that the ADD domain interacts with MeCP2 in cells and that binding of MeCP2 recruits Dnmt3L and Dnmt3a-ADD to pericentromeric heterochromatin. In extrapolation it implies that the heterochromatic localization of Dnmt3a is stabilized by interaction with MeCP2 as well.

### MeCP2 reduces the activity of Dnmt3a in cells

To study the effect of MeCP2 on the DNA methylation activity of Dnmt3a in human cells, the HCT116 DNMT1 hypomorphic cell line was used. This cell line contains a truncated Dnmt1 with reduced activity but active copies of Dnmt3a and Dnmt3b (Egger et al. 2006). Because of the impaired maintenance DNA methylation activity, these cells have an about 20% reduced amount of DNA methylation and show increased levels of hemimethylation. Due to the reduced activity of Dnmt1, the DNA methylation in these cells is more dependent on the activity of Dnmt3a and Dnmt3b, making this cell line a suitable model system to study the effect of the inhibition of Dnmt3a by MeCP2. After generating stable cells lines expressing EYFP-fused MeCP2 or EYFP as control, genomic DNA was isolated and the global levels of 5-methylcytosine were quantified by liquid chromatography-mass spectrometry (LC-MS/MS). As shown in Figure 6, a roughly two-fold decrease in the global methylation of DNA isolated from two independent clones expressing EYFP-MeCP2 was observed. Since Dnmt1 interaction with MeCP2 did not cause a reduction in catalytic activity (Kimura and Shiota, 2003), this result indicates that overexpression of MeCP2 reduces the activity of Dnmt3 enzymes in cells.

### The TRD domain inhibits Dnmt3a2 activity by an allosteric mechanism

It was further aimed to elucidate the mechanism of Dnmt3a inhibition by the TRD domain of MeCP2. Structural studies showed that the ADD domain of Dnmt3a can bind to the catalytic domain of Dnmt3a at an allosteric site, in which Dnmt3a is active, and at an autoinhibitory site, in which the ADD domain blocks DNA interaction and renders Dnmt3a inactive. Binding of the histone H3 N-terminal peptide to the ADD domain of Dnmt3a stabilizes the allosteric conformation and thereby allosterically activates Dnmt3a (e.g. Guo et al. 2015). To investigate the mechanism of the repression of Dnmt3a by the MeCP2 TRD domain, Dnmt3a2 variants containing mutations at Y526 and D531 in the ADD domain, two critical residues involved in the two binding sites at the catalytic domain, were prepared. Y526 mainly contributes to catalytic domain binding in the allosteric conformation and stabilizes the allosteric conformation, while D531 interacts with the catalytic domain in the autoinhibitory conformation and stabilizes the autoinhibitory conformation. The inventors designed mutations that cause large changes of the physio-chemical properties of the corresponding amino acids, in order to selectively disrupt or strongly destabilize one of the two conformations. The Y526E was designed to disrupt the allosteric conformation and D531 R to disrupt the autoinhibitory binding mode. After confirming that both variants still interact with the MeCP2 TRD domain, the inventors investigated if these conformationally locked Dnmt3a variants still respond to the presence of the TRD domain. As shown in Figure 7A, the inhibitory effect of MeCP2 was specifically lost in the D531 R variant, while Y526E was still inhibited. The finding that the D531 R variant, which cannot adopt the autoinhibitory conformation, is not inhibited by the TRD domain suggests that MeCP2 reduces the activity of Dnmt3a by an allosteric mechanism, in which the binding of the MeCP2 TRD domain favors the autoinhibitory conformation of Dnmt3a, leading to inhibition of Dnmt3a.

Previous studies showed that binding of histone H3 to the ADD domain favors the allosteric conformation and activates Dnmt3a (e.g. Guo et al. 2015). To investigate if TRD domain and histone H3 binding to the ADD domain influence each other, pull-down experiments using GST-TRD and Dnmt3a2 in the presence of increasing concentrations of recombinant histone H3 were conducted. As shown in Figure 7B, addition of histone H3 abolished the ADD-TRD interaction, suggesting that binding of H3 and TRD to Dnmt3a is mutually exclusive. Control experiments with histone H4 showed little competition, indicating that the effect was H3 specific. The inventors further conducted DNA methylation experiments with Dnmt3a2 and Dnmt3a2-bound to the unmodified H3 (amino acids 1-19) peptide in the absence and presence of the MeCP2 TRD domain, respectively, and it was observed that the inhibitory effect of the TRD domain was lost in the presence of the H3 peptide (Figure 7C). Hence, binding of H3 to the ADD domain can disrupt the interaction with the TRD domain and relieve the TRD domain-mediated inhibition of Dnmt3a.

Taken together, it was found that MeCP2 has a dual role in the regulation and targeting of Dnmt3a. Binding of MeCP2 to Dnmt3a inactivates the methyltransferase and prevents untargeted enzymatic activity. After association of MeCP2 to active genome parts via binding to hydroxymethylcytosine, Dnmt3a remains inactive, preventing deposition of DNA methylation, because active modifications of the H3 tail, like H3K4 methylation will preclude its binding to the ADD domain. After MeCP2 binding to methylated DNA on a silenced locus, the H3 tail will bind to Dnmt3a, leading to its dissociation from MeCP2 and subsequent DNA methylation. Consequently, more MeCP2-Dnmt3a complexes will get recruited to these sites, generating a positive feedback mechanism that may contribute to the stable maintenance of methylation for example at heterochromatic loci.

### References

Bashtrykov, P.; Rajavelu, A.; Hackner, B.; Ragozin, S.; Carell, T.; Jeltsch, A., Targeted mutagenesis results in an activation of DNA methyltransferase 1 and confirms an autoinhibitory role of its RFTS domain. Chembiochem : a European journal of chemical biology 2014, 15(5):743-748.
Egger, G.; Jeong, S.; Escobar, S.G.; Cortez, C.C.; Li, T.W.; Saito, Y.; Yoo, C.B.; Jones, P.A.; Liang, G., Identification of DNMT1 (DNA methyltransferase 1) hypomorphs in somatic knockouts suggests an essential role for DNMT1 in cell survival. Proceedings of the National Academy of Sciences of the United States of America 2006, 103(38):14080-14085.
Fahy, J.; Jeltsch, A.; Arimondo, P.B., DNA methyltransferase inhibitors in cancer: a chemical and therapeutic patent overview and selected clinical studies. Expert Opin. Ther. Patents 2012, 22(12):1427-1442.
Guo, J.U.; Su, Y.; Shin, J.H.; Shin, J.; Li, H.; Xie, B.; Zhong, C.; Hu, S.; Le, T.; Fan, G.; Zhu, H.; Chang, Q.; Gao, Y.; Ming, G.L.; Song, H., Distribution, recognition and regulation of non-CpG methylation in the adult mammalian brain. Nature neuroscience 2014, 17(2):215-222.
Guo, X.; Wang, L.; Li, J.; Ding, Z.; Xiao, J.; Yin, X.; He, S.; Shi, P.; Dong, L.; Li, G.; Tian, C.; Wang, J.; Cong, Y.; Xu, Y., Structural insight into autoinhibition and histone H3-induced activation of DNMT3A. Nature 2015, 517(7536):640-644.
Hernández, H.G.; Tse, M.Y.; Pang, S.C.; Arboleda, H.; Forero, D.A., Optimizing methodologies for PCR-based DNA methylation analysis. BioTechniques 2013, 55:181-197.
Jeltsch, A. and Lanio, T., Site-directed mutagenesis by polymerase chain reaction. Methods in Molecular Biology 2002, 182:85-94.
Jia, D.; Jurkowska, R.Z.; Zhang, X.; Jeltsch, A.; Cheng, X., Structure of Dnmt3a bound to Dnmt3L suggests a model for de novo DNA methylation. Nature 2007, 449(7159):248-251.
Kimura, H. and Shiota, K., Methyl-CpG-binding protein, MeCP2, is a target molecule for maintenance DNA methyltransferase, Dnmt1. The Journal of biological chemistry 2003, 278(7):4806-4812.
Liu, K.; Liu, Y.; Lau, J.L.; Min, J., Epigenetic targets and drug discovery Part 2: Histone demethylation and DNA methylation. Pharmacology and Therapeutics 2015, 151:121-140.
Patterson, K.; Molloy, L.; Qu, W.; Clark, S., DNA Methylation: Bisulphite Modification and Analysis. Journal of Visualized Experiments : JoVE 2011, 56:3170.
WO 2006/078752 A2
WO 2007/007054 A1
WO 2008/033744 A2
WO 2008/098077 A2
WO 2008/125988 A1
WO 2010/098866 A1
WO 2011/029956 A1
WO 2012/038417 A1
WO 2012/087889 A2

## Claims

1. A DNA methyltransferase inhibitor, or a pharmaceutically acceptable salt thereof, for use in treating Rett syndrome.

2. The DNA methyltransferase inhibitor, or a pharmaceutically acceptable salt thereof, for use according to claim 1, wherein the inhibitor is a nucleoside analogue and/or a non-nucleoside compound.

3. The DNA methyltransferase inhibitor, or a pharmaceutically acceptable salt thereof, for use according to claim 2, wherein the inhibitor is a nucleoside analogue.

4. The DNA methyltransferase inhibitor, or a pharmaceutically acceptable salt thereof, for use according to claim 2 or 3, wherein the nucleoside analogue is selected from the group consisting of
5-azacytidine,
5-aza-2'-deoxycytidine,
5,6-dihydro-5-azacytidine,
5-fluoro-2'-deoxycytidine,
1-(beta-D-ribofuranosyl)-1,2-dihydropyrimidin-2-one,
5-aza-2'-deoxycytidine-p-deoxyguanosine,
fluorocyclopentenylcytosine,
2-(p-nitrophenyl) ethoxycarbonyl-5-aza-2'-deoxycytidine,
CP-4200,
4'-thio-2'-deoxycytidine,
5-aza-4'-thio-2'-deoxycytidine, and
1-beta-D-arabinofuranosyl-5-azacytosine.

5. The DNA methyltransferase inhibitor, or a pharmaceutically acceptable salt thereof, for use according to claim 4, wherein the inhibitor is 5-azacytidine or 5-aza-2'-deoxycytidine.

6. The DNA methyltransferase inhibitor, or a pharmaceutically acceptable salt thereof, for use according to claim 2, wherein the non-nucleoside compound is selected from the group consisting of
hydralazine,
procaine,
mithramycin A,
nanaomycin A,
N-(4-((2-amino-6-methylpyrimidin-4-yl)amino)phenyl)-4-(quinolin-4-ylamino)benzamide,
*N*-phthalyl-L-tryptophan,
*N*-phthalyl-L-tryptophan derivatives,
alkine derivatives,
halomon,
S-adenosyl-L-methionine analogues,
S-adenosyl-L-homocysteine,
S-adenosyl-L-homocysteine analogues,
MG98,
miR-29a,
miR-29c,
Sinefungin,
procainamide,
procainamide derivatives,
procainamide- *N*-phthalyl-L-tryptophan conjugates,
cyclopentathiophene derivatives,
cyclohexathiophene derivatives,
flavone derivatives,
3-nitroflavone derivatives,
flavanones derivatives,
3-chloro-3-nitroflavanone derivatives,
diclone,
laccaic acid,
acridine,
5,5'-Methylenedisalicylic acid,
4-(2-((5-Chloro-2-methoxybenzoyl)amino)ethyl)hydrocinnamic acid,
4-Chloro-N-(4-hydroxy-1-naphthalenyl)-3-nitro-benzenesulfonamide,
(S)-3-(1H-Indol-3-yl)-2-(5-nitro-1,3-dioxo-1,3-dihydro-isoindol-2-yl)-propionic acid,
(R)-2-(1,3-Dioxo-5-phenylethynyl-1,3-dihydro-isoindol-2-yl)-3-(1H-indol-3-yl)-propionic acid,
(S)-2-(2,6-Dioxo-piperidin-1-yl)-3-(1H-indol-3-yl)-propionic acid,
N-hydroxy-4-(2-(4-aminobenzamido)-ethylcarbamoyl)butanamide,
4-amino-N-(2-(ethyl(3-(hydroxyamino)-3-oxopropyl)amino)ethyl)benzamide,
N¹-(2-(4-aminobenzamido)ethyl)-N¹-ethyl-N⁶-hydroxyadipamide,
tetraethylthiuramdisulfide,
(-)-epigallocatechin-3-gallate,
genistein,
curcumin,
psammaplin A, and
psammaplin derivatives.

7. The DNA methyltransferase inhibitor, or a pharmaceutically acceptable salt thereof, for use according to any of claims 1 to 6, wherein the DNA methyltransferase is DNA methyltransferase 3, preferably DNA methyltransferase 3a.

8. A pharmaceutical composition comprising a DNA methyltransferase inhibitor, or a pharmaceutically acceptable salt thereof, and a pharmaceutically acceptable carrier for use in treating Rett syndrome.

9. The pharmaceutical composition for use according to claim 8, wherein the inhibitor is a nucleoside analogue and/or a non-nucleoside compound.

10. The pharmaceutical composition for use according to claim 9, wherein the nucleoside analogue is selected from the group consisting of
5-azacytidine,
5-aza-2'-deoxycytidine,
5,6-dihydro-5-azacytidine,
5-fluoro-2'-deoxycytidine,
1-(beta-D-ribofuranosyl)-1,2-dihydropyrimidin-2-one,
5-aza-2'-deoxycytidine-p-deoxyguanosine,
fluorocyclopentenylcytosine,
2-(p-nitrophenyl) ethoxycarbonyl-5-aza-2'-deoxycytidine,
CP-4200,
4'-thio-2'-deoxycytidine,
5-aza-4'-thio-2'-deoxycytidine, and
1-beta-D-arabinofuranosyl-5-azacytosine.

11. The pharmaceutical composition for use according to claim 9, wherein the non-nucleoside compound is selected from the group consisting of
hydralazine,
procaine,
mithramycin A,
nanaomycin A,
N-(4-((2-amino-6-methylpyrimidin-4-yl)amino)phenyl)-4-(quinolin-4-ylamino)benzamide,
*N*-phthalyl-L-tryptophan,
*N*-phthalyl-L-tryptophan derivatives,
alkine derivatives,
halomon,
S-adenosyl-L-methionine analogues,
S-adenosyl-L-homocysteine,
S-adenosyl-L-homocysteine analogues,
MG98,
miR-29a,
miR-29c,
Sinefungin,
procainamide,
procainamide derivatives,
procainamide- *N*-phthalyl-L-tryptophan conjugates,
cyclopentathiophene derivatives,
cyclohexathiophene derivatives,
flavone derivatives,
3-nitroflavone derivatives,
flavanones derivatives,
3-chloro-3-nitroflavanone derivatives,
diclone,
laccaic acid,
acridine,
5,5'-Methylenedisalicylic acid,
4-(2-((5-Chloro-2-methoxybenzoyl)amino)ethyl)hydrocinnamic acid,
4-Chloro-N-(4-hydroxy-1-naphthalenyl)-3-nitro-benzenesulfonamide,
(S)-3-(1H-Indol-3-yl)-2-(5-nitro-1,3-dioxo-1,3-dihydro-isoindol-2-yl)-propionic acid,
(R)-2-(1,3-Dioxo-5-phenylethynyl-1,3-dihydro-isoindol-2-yl)-3-(1H-indol-3-yl)-propionic acid,
(S)-2-(2,6-Dioxo-piperidin-1-yl)-3-(1H-indol-3-yl)-propionic acid,
N-hydroxy-4-(2-(4-aminobenzamido)-ethylcarbamoyl)butanamide,
4-amino-N-(2-(ethyl(3-(hydroxyamino)-3-oxopropyl)amino)ethyl)benzamide,
N¹-(2-(4-aminobenzamido)ethyl)-N¹-ethyl-N⁶-hydroxyadipamide,
tetraethylthiuramdisulfide,
(-)-epigallocatechin-3-gallate,
genistein,
curcumin,
psammaplin A, and
psammaplin derivatives.

12. An *in vitro* method for diagnosing Rett syndrome in a subject comprising the step of analysing DNA methylation in a sample of the subject.

13. The method according to claim 13, wherein analysing the DNA methylation comprises the steps of
- quantifying the level of DNA methylation in the sample; and
- comparing the level of DNA methylation with a reference value obtained from a reference sample of a healthy individual,
wherein an increased level of DNA methylation in the sample compared to the reference value indicates that the subject has Rett syndrome.

14. The method according to claim 12 or 13, wherein the sample comprises brain cells, preferably neurons.

15. A kit for diagnosing Rett syndrome in a subject *in vitro* comprising reagents for quantifying a level of DNA methylation in a sample of the subject,
wherein an increased level of DNA methylation in the sample compared to a reference value obtained from a reference sample of a healthy individual indicates that the subject has Rett syndrome.


SEQUENCE LISTING

<110> UniversitÃ¤t Stuttgart

<120> DNA methyltransferase inhibitors for Rett syndrome therapy

<130> T30080

<160> 13

<170> BiSSAP 1.3

<210> 1
<211> 20
<212> DNA
<213> Artificial Sequence

<220>
<223> antisense oligodeoxynucleotide MG98

<220>
<221> modified_base
<222> 1
<223> /mod_base="OTHER"
      /note="2'-O-methylthymidine"

<220>
<221> modified_base
<222> 2
<223> /mod_base="OTHER"
      /note="2'-O-methylthymidine"

<220>
<221> modified_base
<222> 3
<223> /mod_base="cm"

<220>
<221> modified_base
<222> 4
<223> /mod_base="OTHER"
      /note="2'-O-methyladenosine"

<220>
<221> modified_base
<222> 17
<223> /mod_base="cm"

<220>
<221> modified_base
<222> 18
<223> /mod_base="cm"

<220>
<221> modified_base
<222> 19
<223> /mod_base="OTHER"
      /note="2'-O-methyladenosine"

<220>
<221> modified_base
<222> 20
<223> /mod_base="cm"

<400> 1
ttnatgtcag ccaaggnnan                                                    20


<210> 2
<211> 22
<212> RNA
<213> Homo sapiens

<220>
<223> hsa-miR-29a-3p

<400> 2
uagcaccauc ugaaaucggu ua                                                 22


<210> 3
<211> 22
<212> RNA
<213> Homo sapiens

<220>
<223> hsa-miR-29c-3p

<400> 3
uagcaccauu ugaaaucggu ua                                                 22


<210> 4
<211> 30
<212> DNA
<213> Artificial Sequence

<220>
<223> unmethylated 30mer upper strand

<400> 4
ttgcactctc ctcccggaag tcccagcttc                                         30


<210> 5
<211> 30
<212> DNA
<213> Artificial Sequence

<220>
<223> unmethylated 30mer lower strand

<400> 5
gaagctggga cttccgggag gagagtgcaa                                         30


<210> 6
<211> 30
<212> DNA
<213> Artificial Sequence

<220>
<223> hemimethylated 30mer upper strand

<400> 6
ttgcactctc ctcccggaag tcccagcttc                                         30

<210> 7
<211> 30
<212> DNA
<213> Artificial Sequence

<220>
<223> hemimethylated 30mer lower strand

<220>
<221> modified_base
<222> 15
<223> /mod_base="m5c"

<400> 7
gaagctggga cttcngggag gagagtgcaa                                        30


<210> 8
<211> 30
<212> DNA
<213> Artificial Sequence

<220>
<223> hemimethylated 30mer with optimized flanks upper strand

<400> 8
gaagctggac agtacgtcaa gagagtgcaa                                        30


<210> 9
<211> 30
<212> DNA
<213> Artificial Sequence

<220>
<223> hemimethylated 30mer with optimized flanks lower strand

<220>
<221> modified_base
<222> 15
<223> /mod_base="m5c"

<400> 9
ttgcactctc ttgangtact gtccagcttc                                        30


<210> 10
<211> 30
<212> DNA
<213> Artificial Sequence

<220>
<223> non-CpG substrate upper strand

<220>
<221> modified_base
<222> 15
<223> /mod_base="m5c"

<400> 10
gaagctggtc cattngatga tggagtgcaa                                        30

<210> 11
<211> 30
<212> DNA
<213> Artificial Sequence

<220>
<223> non-CpG substrate lower strand

<220>
<221> modified_base
<222> 15
<223> /mod_base="m5c"

<400> 11
ttgcactcca tcatngaatg gaccagcttc                                        30


<210> 12
<211> 25
<212> DNA
<213> Artificial Sequence

<220>
<223> forward primer 585mer

<400> 12
gaaggacaac ctgaagtcca ggttg                                             25


<210> 13
<211> 25
<212> DNA
<213> Artificial Sequence

<220>
<223> reverse primer 585mer

<400> 13
gtgtatgacc accagagcct tttgc                                             25